# NOUVEAU FASCICULE DE BREVET EUROPEEN

(11) **EP 2 854 802 B2**
(45) Date de publication et mention de la décision concernant l'opposition: **24.08.2022**
(45) Mention de la délivrance du brevet: 25.07.2018
(21) Numéro de dépôt: 13726504.7
(22) Date de dépôt: 30.05.2013
(51) Int. Cl.: A61K 31/40, A61P 17/02, A61P 17/10, A61P 17/12, A61P 17/16, A61K 8/44, A61K 8/67, A61K 8/81, A61K 8/06

(54) **COMPOSITIONS TOPIQUES, CONTENANT UN RÉTINOÏDE, DE TYPE ÉMULSION HUILE DANS EAU**
TOPISCHE ÖL IN WASSER EMULSIONEN, DIE EIN RETINOID ENTHALTEN
TOPICAL OIL IN WATER EMULSION COMPOSITIONS COMPRISING A RETINOID

(30) Priorité: 01.06.2012 FR 1255094; 01.06.2012 US 201261654729 P
(43) Date de publication de la demande: 08.04.2015
(73) Titulaire: Galderma Research & Development, 06410 Biot (FR)
(72) Inventeur: DUPRAT, Agnès, 06250 Mougins (FR); MALLARD, Claire, 06250 Mougins (FR)
(74) Mandataire: Aera A/S
(86) Numéro de dépôt international: PCT/EP2013/061201
(87) Numéro de publication internationale: WO 2013/178760

(56) Documents cités:
- EP-B1- 1 831 149
- WO-A1-2006/066978
- WO-A1-2008/148968
- WO-A1-2008/152064
- FR-A1- 2 915 682

## Description

L'invention se rapporte à une composition sous forme d'émulsion comprenant, dans un milieu physiologiquement acceptable, au moins un nouveau rétinoïde, l'acide 3"-tert-butyl-4'-(2-hydroxy-ethoxy)-4"-pyrrolidin-1-yl-[1,1';3',1"]-terphenyl-4-carboxylique.

Ces composés, décrits dans le brevet EP1831149, sont de puissants rétinoïdes modulateurs du récepteur nucléaires de l'acide rétinoïque (RAR). Plus spécifiquement du sous-type gamme de ce récepteur (RARy).

Les récepteurs RARs activent la transcription en se liant à des éléments de séquences d'ADN, appelés les éléments de réponse des RAR Element (RARE), sous forme d'un hétérodimère avec les récepteurs X des rétinoïdes (appelés les RXRs).

Trois sous-types de RARs humains ont été identifiés et décrits : les RARα, RARβ et RARγ.

Les récepteurs RAR gamma étant situés dans l'épiderme, il est important que la libération du composé d'acide 3"-tert-butyl-4'-(2-hydroxy-ethoxy)-4"-pyrrolidin-1-yl-[1,1';3',1"]-terphenyl-4-carboxylique s'effectue dans cette partie de la peau pour avoir une efficacité clinique.

Or l'application topique de rétinoïdes peut entraîner une irritation de la peau, une sécheresse et un érythème. De nombreux articles décrivent cet effet irritant comme les articles de Stücker & al. Skin Res Technol. 2002 May;8(2):133-40 ou de Thielitz & al. Am J Clin Dermatol. 2008;9(6):369-81.

Pour obtenir des préparations topiques à usage pharmaceutique contenant des rétinoïdes, de nombreuses techniques sont utilisées, en particulier des émulsions comme par exemple le brevet EP-826366 qui décrit des émulsions pouvant contenir des rétinoïdes ou encore le brevet EP-989846 qui décrit des émulsions contenant des rétinoïdes et au moins un émulsionnant.

Or les émulsionnants sont des molécules faisant partie de la famille chimique des molécules amphiphiles qui sont souvent irritantes. Les compositions sans émulsionnant sont de fait moins irritantes que celles en contenant.

Le fait de ne pas utiliser d'émulsionnant dans les compositions contenant des rétinoïdes permettrait donc de limiter l'irritation cutanée due à la présence de cette classe de molécules.

L'art antérieur décrit des émulsions H/E avec ou sans émulsionnant. On peut citer notamment le brevet US 5,851,538 qui décrit des formulations avec ou sans émulsionnant avec des microsphères poreuses contenant un réseau pratiquement continu de pores ouverts vers l'extérieur et comprenant des rétinoïdes.

Néanmoins, bien que le composé d'acide 3"-tert-butyl-4'-(2-hydroxy-ethoxy)-4"-pyrrolidin-1-yl-[1,';3',1"]-terphenyl-4-carboxylique possède des propriétés de stabilité chimiques et physiques intéressantes pour des préparations à usages pharmaceutiques, ils se dégradent chimiquement dans nombreux de leurs solvants.

Il existe par conséquent un besoin d'avoir des compositions pharmaceutiques stables et bien tolérées, contenant le composé d'acide 3"-tert-butyl-4'-(2-hydroxy-ethoxy)-4"-pyrrolidin-1-yl-[1,1';3',1"]-terphenyl-4-carboxylique.

Un premier objet selon l'invention concerne une composition de type émulsion huile dans eau comprenant
- Une phase grasse comprenant :
   l'acide 3"-tert-butyl-4'-(2-hydroxy-ethoxy)-4"-pyrrolidin-1-yl-[1,1';3',1"]-terphenyl-4-carboxylique;
   au moins un solvant principal de l'acide 3"-tert-butyl-4'-(2-hydroxy-ethoxy)-4"-pyrrolidin-1-yl-[1,1';3',1"]-terphenyl-4-carboxylique, choisi parmi l'alcool benzylique, le laureth-4, le phénoxyéthanol, le propylène glycol monocaprylate, le pentylène glycol, le dimethyl isosorbide, et leurs mélanges, et au moins une huile co-solvante de l'acide 3"-tert-butyl-4'-(2-hydroxy-ethoxy)-4"-pyrrolidin-1-yl-[1,1';3',1"]-terphenyl-4-carboxylique, choisie parmi les triglycérides caprylique/caprique, l'huile d'amande douce, le monocaprylate de propylène glycol, le laurate de propylène glycol, le sesquioléate de sorbitan, l'adipate de diisopropyle, l'éther de PPG-15 stéaryle, l'ester de PEG-6 et huile de noyau d'abricot, et leurs mélanges, et
- Une phase aqueuse comprenant au moins un agent gélifiant, choisi parmi les polymères d'origine végétale, les gommes, les pectines, la cellulose et ses dérivés, les polymères d'origine microbiologiques tel que la gomme xanthane, et les polymères gélifiants d'origine synthétique.

Un second objet selon l'invention concerne une composition telle que décrite ci-dessus pour son utilisation en tant que médicament.

Un troisième objet selon l'invention concerne une composition telle que décrite ci-dessus pour son utilisation dans le traitement des pathologies telles que :
1) les affections dermatologiques liées à un désordre de la kératinisation portant sur la différenciation et sur la prolifération cellulaire notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle ;
2) les troubles de la kératinisation, notamment les ichtyoses, les états ichtyosiformes, l'ichtyose lamellaire, la maladie de Darrier, les kératodermies palmoplantaires, les leucoplasies, le pityriasis rubra pilaire et les états leucoplasiformes, le lichen cutané ou muqueux (buccal) ;
3) les affections dermatologiques avec une composante immuno-allergique inflammatoire, avec ou sans trouble de la prolifération cellulaire, et notamment toutes les formes de psoriasis, qu'il soit cutané, muqueux ou unguéal, et même le rhumatisme psoriasique, ou encore la dermatite atopique et les différentes formes d'eczema;
4) les désordres cutanés dus à une exposition aux rayonnements U.V. ainsi que pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique, telle la xérose, les pigmentations et les rides ;
5) Toute condition liée à des proliférations dermiques ou épidermiques bénignes, qu'elles soient ou non d'origine virale telles que verrues vulgaires, les verrues planes , le molluscum contagiosum et l'épidermodysplasie verruciforme, les papillomatoses orales ou florides,;
6) les désordres dermatologiques tels que les dermatoses immunes comme le lupus érythémateux, les maladies immunes bulleuses et les maladies du collagène, telle la sclérodermie ;
7) les stigmates de l'atrophie épidermique et/ou dermique induite par les corticostéroïdes locaux ou systémiques, ou toute autre forme d'atrophie cutanée,
8) les troubles de la cicatrisation, ou pour prévenir ou pour réparer les vergetures, ou encore pour favoriser la cicatrisation,
9) toute affection d'origine fongique au niveau cutané tel que le tinea pedis et le tinea versicolor,
10) les désordres de la pigmentation, tel l'hyperpigmentation, le mélasma, l'hypopigmentation ou le vitiligo ;
11) les états cancéreux ou précancéreux, cutanés ou muqueux comme les kératoses actiniques, la maladie de Bowen, les carcinomes in-situ, le kératoacanthome et les cancers cutanés comme le carcinome basocellulaire (BCC), le carcinome spinocellulaire (SCC) et les lymphomes cutanés tel que le lymphome T .

Un quatrième objet selon l'invention concerne un procédé de préparation d'une composition de type émulsion huile dans eau telle que décrite précédemment et comprenant les étapes suivantes :
a) Solubilisation des excipients hydrophiles sous agitation
b) solubilisation sous agitation, du rétinoïde dans le solvant principal
c) Ajout des excipients lipophiles
d) Gélification de la phase aqueuse en ajoutant l'agent gélifiant
e) Ajout de la phase huileuse, puis de l'huile de silicone

L'invention sera décrite plus en détail dans la description et les exemples qui suivent, ainsi que dans les figures annexées dans lesquelles :
La Figure 1 présente le profil de distribution dans les différents compartiments de la peau d'une composition selon l'invention.
La Figure 2 présente la cinétique de pénétration dans l'épiderme d'une composition selon l'invention.
La Figure 3 présente les résultats d'une étude de tolérance d'une composition selon l'invention par rapport à un gel de référence.

### Description détaillée de l'invention

A des fins de facilité de lecture, on considèrera le composé A dans la suite du texte comme étant décrits comme il suit :
Composé A: comme étant l'acide 3"-tert-butyl-4'-(2-hydroxy-ethoxy)-4"-pyrrolidin-1-yl-[1,1';3',1"]-terphenyl-4-carboxylique

Connaissant les caractéristiques physico-chimiques de l'actif, la Demanderesse a dû faire face à un certain nombre de contraintes de mise en œuvre du composé A.

En effet le composé A:
- est soluble dans peu de solvants habituellement utilisés dans les phases grasses des émulsions topiques.
- se dégrade chimiquement dans nombreux de ses solvants.
- se dégrade chimiquement en présence de nombreux émulsionnants.

Un premier objet selon la présente invention décrit des compositions contenant au moins un composé comprenant le composé A sous la forme d'émulsions de type H/E (Huile dans Eau) et dans lesquelles l'actif est solubilisé dans la phase grasse.
Selon un mode de réalisation particulièrement préféré, la composition selon l'invention ne contient pas d'agent émulsionnant.
Ces émulsions présentent une bonne stabilité physique et chimique, une vitesse de pénétration rapide et un niveau de pénétration élevé dans l'épiderme et/ou le derme.

Selon un mode de réalisation particulièrement préféré, la composition selon l'invention ne contient pas d'agent émulsionnant.

Ces émulsions présentent une bonne stabilité physique et chimique, une vitesse de pénétration rapide et un niveau de pénétration élevé dans l'épiderme et/ou le derme.

Par émulsion on entend un mélange macroscopiquement homogène mais microscopiquement hétérogène, de deux substances liquides non miscibles que l'on appellera phases. Une émulsion H/E (Huile dans eau) est composée d'une phase grasse (ou huileuse) dispersée dans une phase aqueuse. Dans l'invention, les compositions contiennent l'actif décrit par le composé A à des concentrations allant de 0.00001% à 1% en poids préférentiellement de 0.0001 à 0.1 % en poids et plus préférentiellement de 0.001 à 0.1 % en poids par rapport au poids total de la composition.

Dans l'invention, les compositions contiennent au moins un agent gélifiant choisi parmi les polymères d'origine végétale, les gommes, les pectines, la cellulose et ses dérivés, les polymères d'origine microbiologiques tel que la gomme xanthane, et les polymères gélifiants d'origine synthétique.

Par agent gélifiant, on entend un composé polymère apte à conférer à la composition la texture d'un gel.

A titre d'exemple non limitatif d'agents gélifiants pouvant entrer dans les compositions, on peut citer l'Acryla-tes/C10-30 Alkyl Acrylate Crosspolymer vendu sous le nom de Pemulen TR-1 ou Pemulen TR-2 par la société Lubrizol, les gélifiants de la famille des polyacrylamides comme le mélange Sodium acrylamide/acryloyldimethyltaurate copolymer / isohexadecane / polysorbate 80 vendu sous le nom de Simulgel 600PHA par la société SEPPIC, le mélange polyacrylamide/isoparaffine C13-14/laureth-7 vendu sous le nom de Sepigel 305 par la société SEPPIC,
les carbomers vendus sous le nom d'Ultrez 20^{®}, d'Ultrez 10^{®}, de Carbopol 1382^{®} ou de Carbopol ETD2020NF^{®}, de Carbopol 981 ou encore Carbopol 980 par la Société Lubrizol, les polysaccharides avec à titre d'exemples non limitatifs la gomme de xanthane telle que le Xantural 180^{®} vendue par la société Kelco, la gellan gum vendue sous le nom de Kelcogel par la société Kelco, la gomme guar, la cellulose et ses dérivés tel que la microcristalline cellulose et carboxymethyl cellulose de sodium vendues sous le nom d'Avicel CL-611 par la société FMC Biopolymer, l'hydroxypropylmethylcellulose en particulier le produit vendu sous le nom de Methocel E4M premium par la société Dow Chemical ou l'hydroxyéthylcellulose, en particulier, le produit vendu sous le nom de Natrosol HHX 250^{®} par la société Ashland, la sodium carboxymethylcellulose en particulier la Blanose cellulose gum 7F vendu par la société Ashland, la famille des aluminium magnésium silicates tel que le Veegum K vendu parla société Vanderbilt, la famille des polymères acryliques couplés à des chaînes hydrophobes tel que le PEG-150/decyl/SMDI copolymer vendu sous le nom de Aculyn 44 (polycondensat comprenant au moins comme éléments, un polyéthylèneglycol à 150 ou 180 moles d'oxyde d'éthylène, de l'alcool décylique et du méthylène bis(4-cyclohexylisocyanate) (SMDI), à 35% en poids dans un mélange de propylèneglycol (39%) et d'eau (26%)), la famille des amidons modifiés tels que l'amidon de pomme de terre modifié vendu sous le nom de Structure Solanacée ou bien leurs mélanges, la famille des carraghénanes en particulier réparties sous quatre grandes familles : κ, λ, β, ω tel que les Viscarin^{®} et les Gelcarin^{®} commercialisés par la société IMCD

De façon préférentielle un agent gélifiant de type polyacrylamide comme le Simulgel 600 PHA^{®}, qui a des propriétés épaississantes et stabilisantes, est utilisé aux concentrations allant de 0,005 à 5 % en poids et préférentiellement, allant de 1% à 4% en poids.

L'homme de l'art sait qu'habituellement pour un système d'émulsifiant, la proportion d'émulsifiant nécessaire pour émulsionner une phase grasse dans une émulsion huile dans eau et de l'ordre de 1/5 du % de la phase grasse. A titre d'exemple non limitatif, une phase grasse représentant au minimum 11% des ingrédients des formules nécessiterait au minimum 2,2% d'émulsionnants.

Selon un mode de réalisation préféré de l'invention, aucun agent émulsionnant n'est utilisé. Il est malgré tout possible que certains ingrédients contiennent de faibles pourcentages d'agent émulsionnant dans leur propre composition. Au vu du faible pourcentage qui peut en résulter dans la composition selon l'invention, ils ne peuvent pas jouer le rôle de d'agent émulsionnant de la composition (teneur de préférence inférieure à 0.6% en poids par rapport au poids total de la composition).

Dans l'invention, les compositions contiennent une phase grasse composée:
- d'un solvant principal d'un actif décrit par le composé A choisi parmi l'alcool benzylique, le laureth-4, le phénoxyéthanol, le propylène glycol monocaprylate, le pentylène glycol ou le dimethyl isosorbide, et préférentiellement le phénoxyéthanol vendu par exemple sous le nom de phénoxétol par Clariant.
   Lorsque le solvant principal est la phénoxyéthanol la quantité de phénoxyéthanol va de 0.2 à 5% en poids et préférentiellement de 0.5 à 2% en poids par rapport au poids total de la composition.
   Par solvant principal on entend un liquide qui a la propriété de dissoudre, de diluer ou d'extraire d'autres substances sans provoquer de modification chimique de ces substances et sans lui-même se modifier.
   Selon l'invention, un solvant principal est un tel liquide, dans lequel le composé A présentent une solubilité, à température ambiante et pression atmosphérique, supérieure ou égale à 0,1% en poids.
- d'une ou plusieurs huiles co-solvantes d'un actif décrit par le composé A choisies parmi les huiles co-solvantes suivantes : Caprylic/ capric triglycérides (Miglyol 812N) fourni par IMCD, Prunus Amygdalus Dulcis (Sweet Almond) oil (Huile d'amande douce) fournie par SICTIA, Propylène glycol monocaprylate (Capryol 90) fourni par GATTEFOSSE, Propylène glycol laurate (Lauroglycol FCC) fourni par GATTEFOSSE, Sorbitan Sesquioleate (Arlacel 83VPharma) fourni par CRODA, Diisopropyl Adipate (Crodamol DA) fourni par CRODA, PPG-15 stearyl ether (Arlamol PS15E-LQ) fourni par CRODA, l'ester de PEG-6 et huile de noyau d'abricot (Apricot Kernel Oil PEG-6 Ester, Labrafil M1944CS) fourni par GATTEFOSSE à des taux pouvant aller de 0.5 à 50% en poids et préférentiellement de 4 à 30% en poids.
Par co-solvant on entend une substance ayant un rôle de solvant en association avec une autre substance.

Dans l'invention, les compositions décrites ci-dessus peuvent en outre contenir des additifs (parmi lesquels on peut citer les catégories suivantes, utilisés seuls ou en combinaison):
- Une ou plusieurs huiles de silicone améliorant les propriétés de la formule à l'application, comme la Cyclomethicone (St-Cyclomethicone 5NF) ou la Dimethicone (Q7 9120 silicon fluid de viscosité de 20 est à 12500 est de Dow Corning) entre 0 et 10% et préférentiellement entre 0 et 4%.
- Un ou plusieurs agents conservateurs, tels que le méthyl parabène, le propyl parabène, le chlorure de benzalkonium, le phénoxyéthanol vendu sous le nom de phénoxétol par Clariant, l'alcool benzylique vendu sous le nom d'alcool benzylique par Merck, le sorbate de potassium vendu sous le nom de Sorbate de potassium par VWR, l'acide benzoique vendu sous le nom Acide benzoïque par VWR, le 2-Bromo-2-Nitropropane-1,3-Diol vendu sous le nom de Bronopol par Jan Dekker International la Chlorhexidine vendu sous le nom de Chlorhexidine di gluconate 20% solution par Arnaud Pharmacie, le chlorocrésol et ses dérivés, le sodium benzoate vendu sous le nom de Probenz SP par la société Unipex, l'alcool éthylique et la diazolidinylurée. Ces conservateurs peuvent être utilisés seul ou en association afin de protéger efficacement les formules contre toute contamination bactérienne, en des teneurs allant de 0% à 5% en poids et préférentiellement de 0.01 à 2% en poids.

Par agent conservateur on désigne toute substance capable de s'opposer aux altérations d'origine chimique ou microbiologique d'un produit.
- De l'éthanol dont la quantité peut être comprise entre 0 et 30% en poids et préférentiellement entre 0 et 10% en poids.
- Des agents humectants préférentiellement des polyols et de préférence sélectionnés parmi le propylène glycol, la glycérine, la diglycerine ou le sorbitol (Neosorb fourni par ROQUETTE, Parteck SI fourni par Merck mais aussi Sorbitol USP Powder fourni par LIPO CHEMICALS) et dont la quantité est comprise entre 0 et 40% en poids par rapport au poids total de la composition et préférentiellement entre 5 et 35%.
- Des agents chélatant comme l'EDTA (ethylenediaminetetraacetic acid) et ses dérivés ou sels, la dihydroglycérine, les acides citriques et tartriques, la gluconolactone vendu sous le nom glucono delta lactone SG par Jungbunzlauer ou des mélanges de ceux-ci.
- Des antioxydants tel que la vitamine E et ses dérivés, comme le DL alpha Tocophérol ou l'acétate de tocophérol de Roche ; la vitamine C et ses dérivés, comme l'Ascorbyl Palmitate de Roche, le Butylhydroxy toluène vendu sous le nom de Nipanox BHT par Clariant.
- Des agents apaisants et/ou anti-irritants tels que le PPG-12/SMDI copolymer vendu par la société Bertek pharmaceuticals sous le nom commercial de Polyolprepolymer-2, l'acide glycyrrhétinique ou ses dérivés comme par exemple l'Enoxolone vendu par la société BASF, l'acide hyaluronique tel quel ou sous sa forme hyaluronate de sodium vendu sous le nom commercial de HYAL. NA PWD PH 15-51-45 par la société Contipro, l'allantoine vendue sous le nom de RONACARE ALLANTOINE par MERCK.
- Tout autre additif usuellement utilisé dans le domaine pharmaceutique et cosmétique permettant de conférer à la dite préparation des propriétés spécifiques.

### Composition générale pour l'acné :

Lorsqu'elle est destinée au traitement de l'acné, la composition selon l'invention contient avantageusement les ingrédients suivants, les pourcentages étant exprimés en poids par rapport au poids total de la composition de type émulsion huile dans eau :
- de 0.00001% à 1% et préférentiellement de 0.0001 à 0.1% du composé A
- de 0.005 à 10 % et préférentiellement de 1 à 5% d'agent gélifiant
- de 0.2 à 5% et préférentiellement de 0.5 à 2% de solvant principal du composé A
- de 0.5 à 50% et préférentiellement de 4 à 15% d'huiles co-solvantes du composé A
- de 0 à 20% et préférentiellement de 0 à 5% d'huiles minérales
- de 0 à 50% et préférentiellement de 5 à 35% de polyol
- de 0 à 10% et préférentiellement 0 à 4% d'huile de silicone
- de 0 à 5% et préférentiellement de 0.01 à 2% de système conservateur
- de 0 à 30% et préférentiellement de 0 à 10% d'éthanol
- de 0 à 15% et préférentiellement de 0.1 à 10% d'additifs

### Compositions adaptées à l'ichtyose, l'hyperkératose palmoplantaire ou au psoriasis :

Dans ce cas, la composition selon l'invention contient avantageusement les ingrédients suivants, les pourcentages étant exprimés en poids par rapport au poids total de la composition de type émulsion huile dans eau :
- de 0.00001% à 1% et préférentiellement de 0.0001 à 0.1% du composé A
- de 0.005 à 10 % et préférentiellement de 1 à 5% d'agent gélifiant
- de 0.2 à 5% et préférentiellement de 0.5 à 2% de solvant principal du composé A
- de 0.5 à 50% et préférentiellement de 10 à 30% d'huiles co-solvantes du composé A
- de 1 à 50% et préférentiellement de 10 à 30% de polyol
- de 0 à 10% et préférentiellement 0 à 4% d'huile de silicone
- de 0 à 5% et préférentiellement de 0.01 à 2% de système conservateur
- de 0 à 15% et préférentiellement de 0.1 à 10% d'additifs

Un autre objet selon l'invention concerne un procédé de préparation d'une composition décrite telle que précédemment et comprenant les étapes suivantes :

### A) Préparation de la phase aqueuse

Solubilisation des excipients hydrophiles sous agitation, si nécessaire à chaud

### B) Préparation de la phase huileuse

Dans un récipient adéquat, solubilisation sous agitation, du composé A dans le Phénoxyéthanol, si nécessaire à chaud.

Laisser revenir à Température Ambiante et ajouter les excipients lipophiles sauf l'huile de silicone (par exemplele ST-cyclomethicone 5) lorsque celle-ci est présente.

### C) Mélange des deux phases.

A température ambiante, gélifier la phase aqueuse en ajoutant l'agent gélifiant (par exemple le Simulgel 600PHA), ajouter ensuite la phase huileuse, puis l'huile de silicone lorsque celle-ci est présente.

### Exemples

### Exemple 1 - Pré-formulation

Afin de réaliser une émulsion huile dans eau contenant le composé A dans la phase grasse, des études de préformulations ont été réalisées afin de mettre en évidence les excipients permettant une bonne solubilisation ainsi qu'une bonne stabilité de l'actif.

### (1) Liste des excipients de phase grasse dans lesquels la solubilité maximale a été déterminée par HPLC :

| **Excipients** | | **Solubilité maximale** |
|---|---|---|
| **Nom commercial** | **Nom INCI** | **%** |
| Alcool benzylique | Benzyl Alcohol | 2,388 |
| Brij 30 | Laureth-4 | 2,03 |
| Phénoxétol | Phenoxyethanol | 1,957 |
| Capryol 90 | Propylène glycol monocaprylate | 0,802 |
| Hydrolite 5P | Pentylène glycol | 0,482 |
| Arlasolve DMI | Dimethyl Isosorbide | 0,400 |
| Crodamol IPM | Isopropyl myristate | < 0,1 |

La limite, en dessous de laquelle le composé A est considéré comme non solubilisé, est de 0,1% en poids.

### (2) Stabilité du composé A dans ses principaux solvants:

Ces études de stabilité du composé A dans ses principaux solvants montrent que le composé A se dégrade chimiquement dans nombreux de ses solvants.

Ces résultats nous ont permis de sélectionner notre solvant principal (phénoxyéthanol) et nos huiles co-sol-vantes parmi les huiles solvantes qui présentent de bons résultats de stabilité, ceci dans l'objectif de développer des émulsions H/E dans lesquelles le composé A est solubilisé dans la phase huileuse.

| **Excipients** | | **COMPOSÉ A** | **Résultats de stabilité** |
|---|---|---|---|
| **Nom commercial** | **Nom INCI** | **%** | |
| Miglyol 812N | Caprylic/capric triglycérides | 0,005% | Stable/ Ok 6Mois 40°C |
| Huile d'amande douce | Prunus Amygdalus Dulcis (Sweet Almond) Oil | 0,005% | Stable/ Ok 6Mois 40°C |
| Capryol 90 | Propylène glycol monocaprylate | 0,05% | Stable/ Ok 6Mois 40°C |
| Arlacel 83V Pharma | Sorbitan Sesquioleate | 0,05% | Stable/ Ok 6Mois 40°C |
| Crodamol DA | Diisopropyl Adipate | 0,05% | Stable/ Ok 6Mois 40°C |
| Lauroglycol FCC | Propylène glycol laurate | 0,05% | Stable/ Ok 6Mois 40°C |
| Arlamol PS15E-LQ | PPG-15 stearyl ether | 0,05% | Stable/ Ok 6Mois 40°C |
| Phénoxétol | Phenoxyethanol | 0,05% | Stable/ Ok 6Mois 40°C |
| Labrafil M1944CS | Apricot Kernel Oil PEG-6 Ester | 0,05% | Stable/ Ok 6Mois 40°C |
| Dipropylène Glycol Care | Dipropylène glycol | 0,05% | Instable |
| Brij 30 | Laureth-4 | 0,05% | Instable |
| Alcool benzylique | Benzyl Alcohol | 0,05% | Instable |
| Eutanol G | Octyldodecanol | 0,05% | Instable |
| Myritol PC | Propylène glycol dicaprylate/ dicaprate | 0,05% | Instable |
| Arlasolve DMI | Dimethyl Isosorbide | 0,05% | Instable |
| Marcol 152 | Paraffinum Liquidum | 0,05% | N/A |

### (3) Stabilité du composé A dans des mélanges d'excipients (solvant/ tensio-actifs) déterminée par HPLC :

Des études de stabilité du composé A solubilisé dans des huiles (dans lesquelles il est stable) en présence de surfactants ont été réalisées :

| **Mélange excipients** | | **COMPOSÉ A** | **Résultats stabilité** |
|---|---|---|---|
| **Nom commercial** | **Nom INCI** | **%** | |
| Simulsol M45/ Crodamol DA | PEG-8 Stearate/ Diisopropyl Adipate | 0,05% | Instable |
| Cremophor EL/ Labrafil M1944CS | PEG-35 Castor Oil/ Apricot Kernel Oil PEG-6 Ester | 0,05% | Instable |
| Tween 80/Arlamol PS15E-LQ | Polysorbate-80/ PPG-15 stearyl ether | 0,05% | Instable |
| Cremophor EL/ Lauroglycol FCC | PEG-35 Castor Oil/ Propylène glycol laurate | 0,05% | Instable |
| Tween 80/ Hexylene glycol | Polysorbate-80/ Hexylene glycol | 0,2% | Instable |
| Cremophor EL/ Labrafil M1944CS | PEG-35 Castor Oil/ Apricot Kernel Oil PEG-6 Ester | 0,2% | Instable |
| Cremophor RH40/ Crodamol DA | PEG-40 Hydrogenated Castor Oil/ Diisopropyl Adipate | 0,2% | Instable |
| Tween 80/ Lauroglycol FCC | Polysorbate-80/ Propylène glycol laurate | 0,2% | Instable |
| Simulsol M45/ Crodamol DA | PEG-8 Stearate/ Diisopropyl Adipate | 0,2% | Instable |
| Arlacel 165/ Lauroglycol FCC | Glyceryl Stearate PEG-100 Stearate/ Propylène glycol laurate | 0,05% | Instable |
| GlucateSS-Glucamate SSE-20/Arlamol PS15E-LQ | Methyl Glucose Sesquistearate-PEG-20 | 0,05% | Instable |
| | Methyl Glucose Sesquistearate/ PPG-15 stearyl ether | | |
| Brij 721/ Arlamol PS15E-LQ | Steareth-21/ PPG-15 stearyl ether | 0,05% | Stable/ Ok 3Mois 40°C |
| Brij 721/ Lauroglycol FCC | Steareth-21/ Propylène glycol laurate | 0,05% | Stable/ Ok 3Mois 40°C |
| Eumulgin B2/ Arlamol E | Ceteareth-20/ PPG-15 stearyl ether | 0,05% | Stable/ Ok 3Mois 40°C |
| Arlacel 165/ Arlamol E | Glyceryl Stéarate PEG-100 Stearate/ PPG-15 stearyl ether | 0,05% | Stable/ Ok 3Mois 40°C |
| GlucateSS-Glucamate SSE-20/ Lauroglycol FCC | Methyl Glucose Sesquistearate-PEG-20 | 0,05% | Stable/ Ok 3Mois 40°C |
| | Methyl Glucose Sesquistearate/ Propylène glycol laurate | | |

Les limites fixées pour une bonne stabilité sont 95%-105% en pourcentage relatif par rapport au T0.

Ces études ont montré que le composé A se dégrade chimiquement en présence de nombreux surfactants. Suite à ces résultats, nous avons donc souhaité développer une émulsion H/E sans émulsionnant.

### Exemple 2 : Formulations

Dans les exemples suivants, les formules sont caractérisées à T0. La stabilité physique et chimique des formulations est réalisée après conservation à température ambiante (TA) et +40°C après T+1Mois et/ou T+2Mois ou T+3Mois ou T+6Mois. Le matériel et les méthodes utilisés pour ces caractérisations sont décrits ci-dessous.

Le composé A est défini comme étant l'acide 3"-tert-butyl-4'-(2-hydroxy-ethoxy)-4"-pyrrolidin-1-yl-[1,1';3',1"]-terphenyl-4-carboxylique.

### -Dosage chimique du composé A:

- Matériel: HPLC
- Expression des résultats: le titre de l'actif est exprimé en % relatif par rapport au % initial effectué à T0. Les limites fixées pour une bonne stabilité sont 95%-105%

### -Observation macroscopique:

- L'observation macroscopique permet de garantir l'intégrité physique des produits à T0 et après stabilité.

### -Observation microscopique:

- L'observation microscopique permet d'évaluer la bonne solubilisation du composé A dès T0, la non recristallisation au cours du temps ainsi que la taille des globules de la phase huileuse.
- Matériel: Microscope AXIO ZEISS

### -pH:

- Matériel: pH mètre METTLER TOLEDO Seven Multi
- Méthode : Mesures effectuées à température ambiante après stabilisation 24h dans une enceinte à 25°C de tous les échantillons.

### -Viscosité:

- La mesure de la viscosité permet d'évaluer la consistance des formules réalisées.
- Matériel: Brookfield RV DVII + Pro
- Méthode: Mesures effectuées à température ambiante après stabilisation 24h dans une enceinte à 25°C de tous les échantillons. La valeur est lue après 1 minute. Le choix du mobile et de la vitesse seront décrits dans chaque exemple de composition. Les valeurs obtenues sont exprimées en centipoises (Cps).

### -Centrifugation:

- La centrifugation permet d'évaluer la résistance des formules à une contrainte mécanique.
- Matériel: Galaxy 14D VWR
- Méthode: 30 minutes à 5000 rpm
- Un résultat conforme signifie qu'il n'y a ni séparation de phases, ni exudat.

### Formule 1

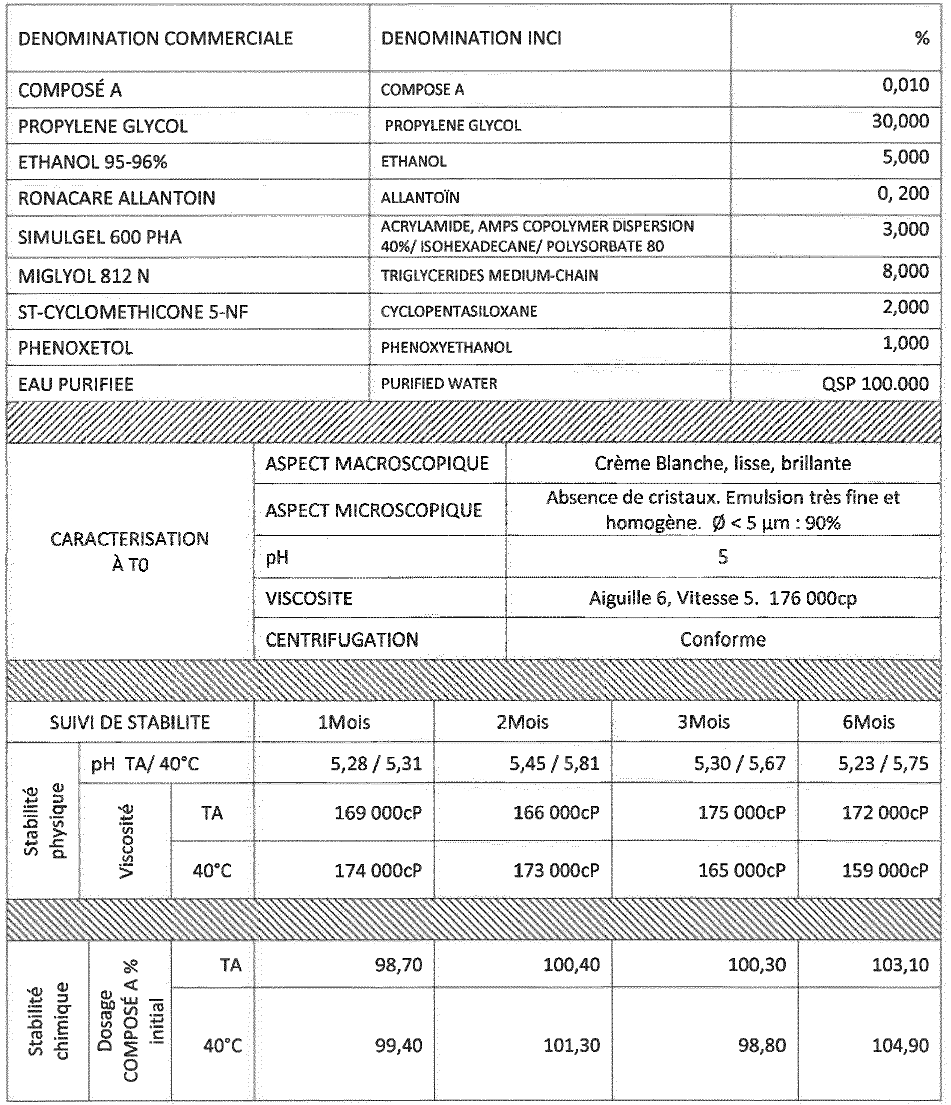

Ces résultats montrent une bonne stabilité physique et chimique de l'actif et de la composition dans son ensemble dans le temps.

### Formule 2

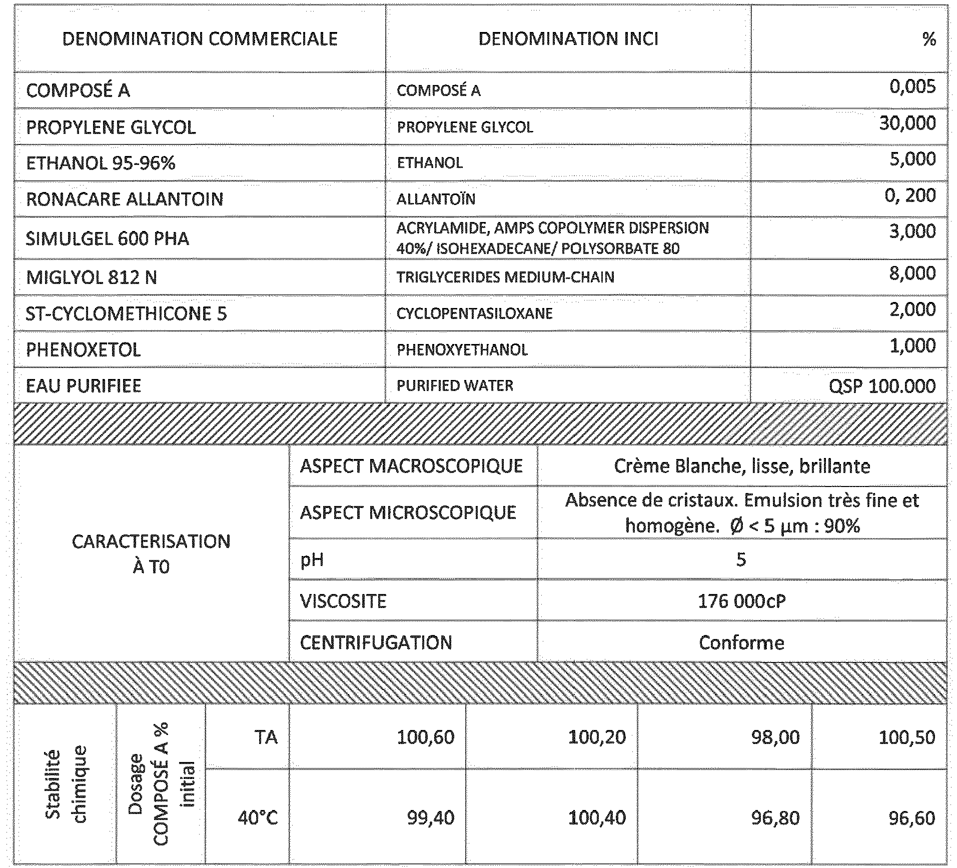

Ces résultats montrent une bonne stabilité physique et chimique de l'actif et de la composition dans son ensemble dans le temps.

### Formule 3

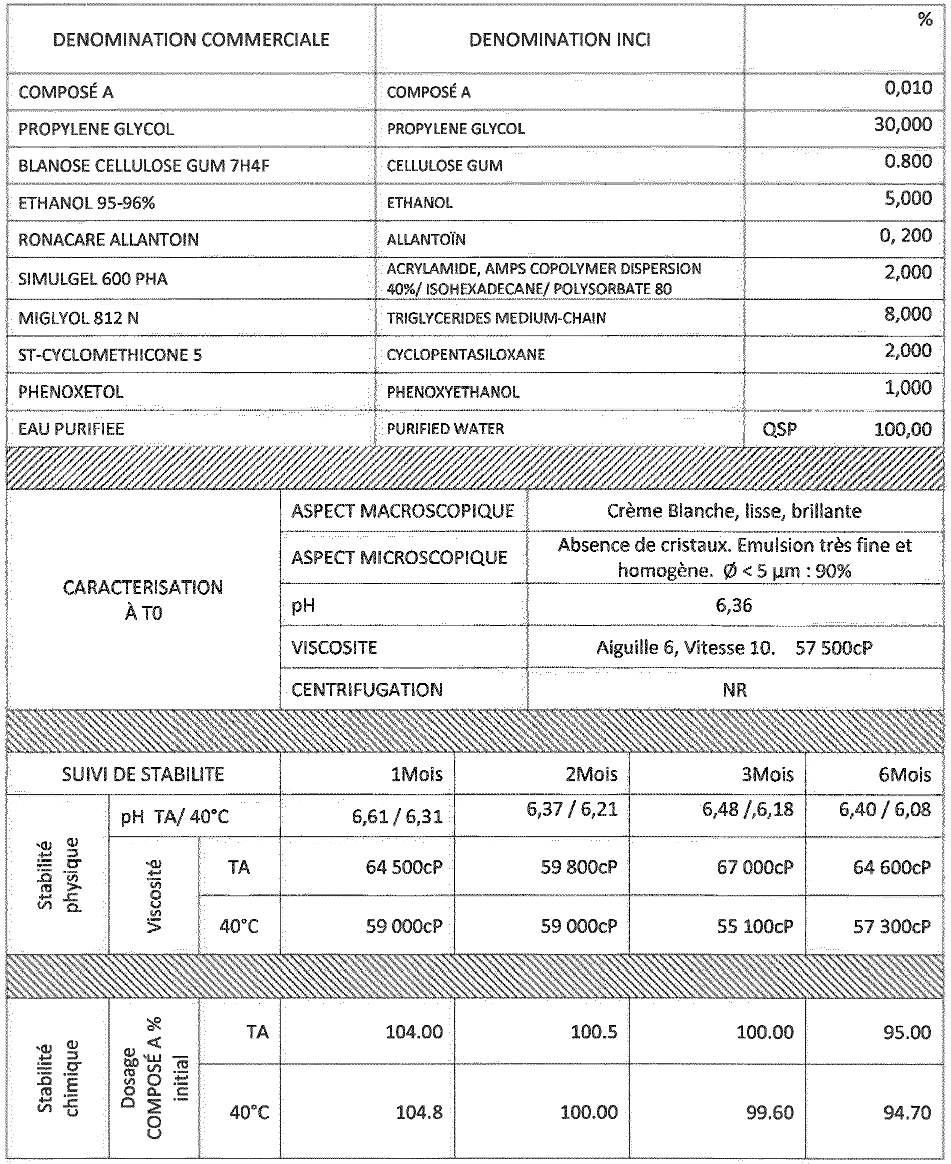

Ces résultats montrent une bonne stabilité physique et chimique de l'actif et de la composition dans son ensemble dans le temps.

### Formule 4

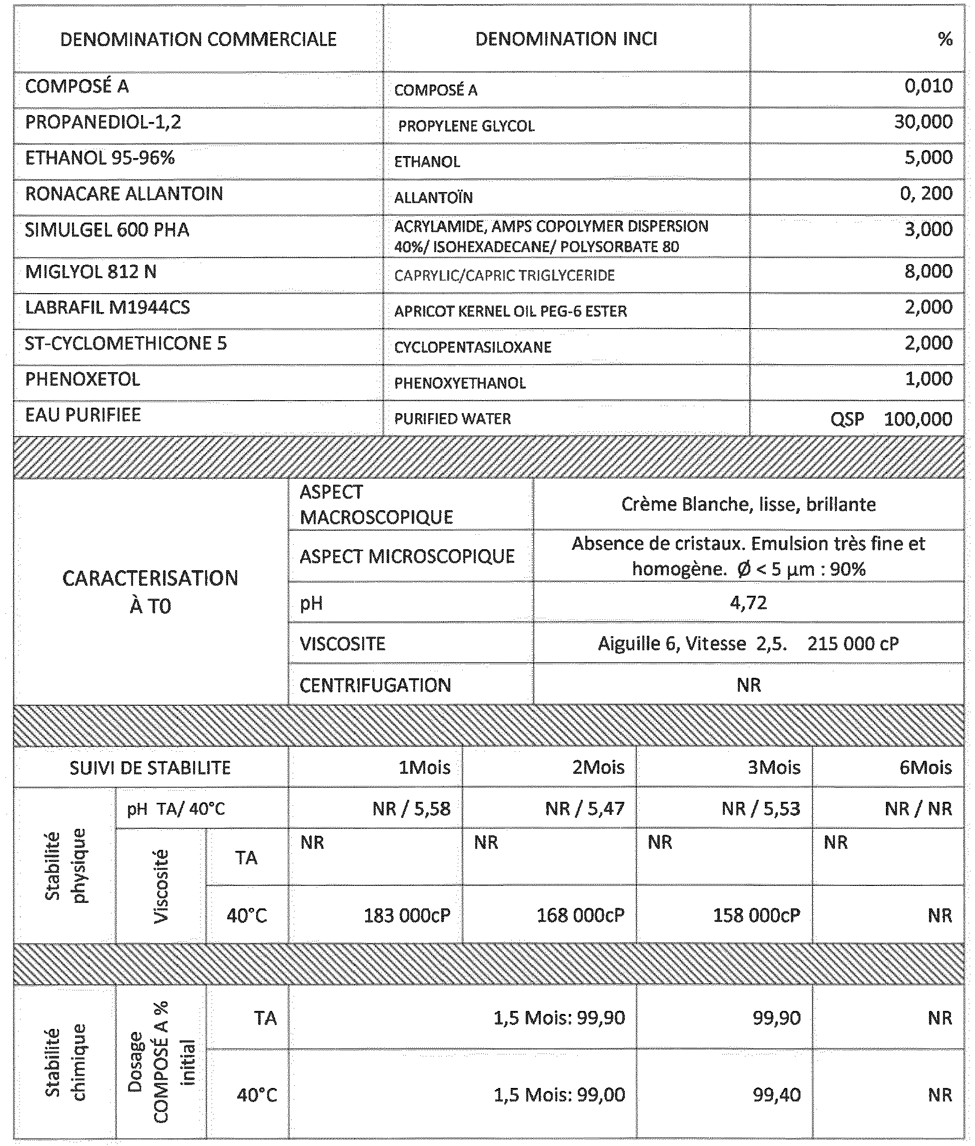

Ces résultats montrent une bonne stabilité physique et chimique de l'actif et de la composition dans son ensemble dans le temps.

### Formule 5

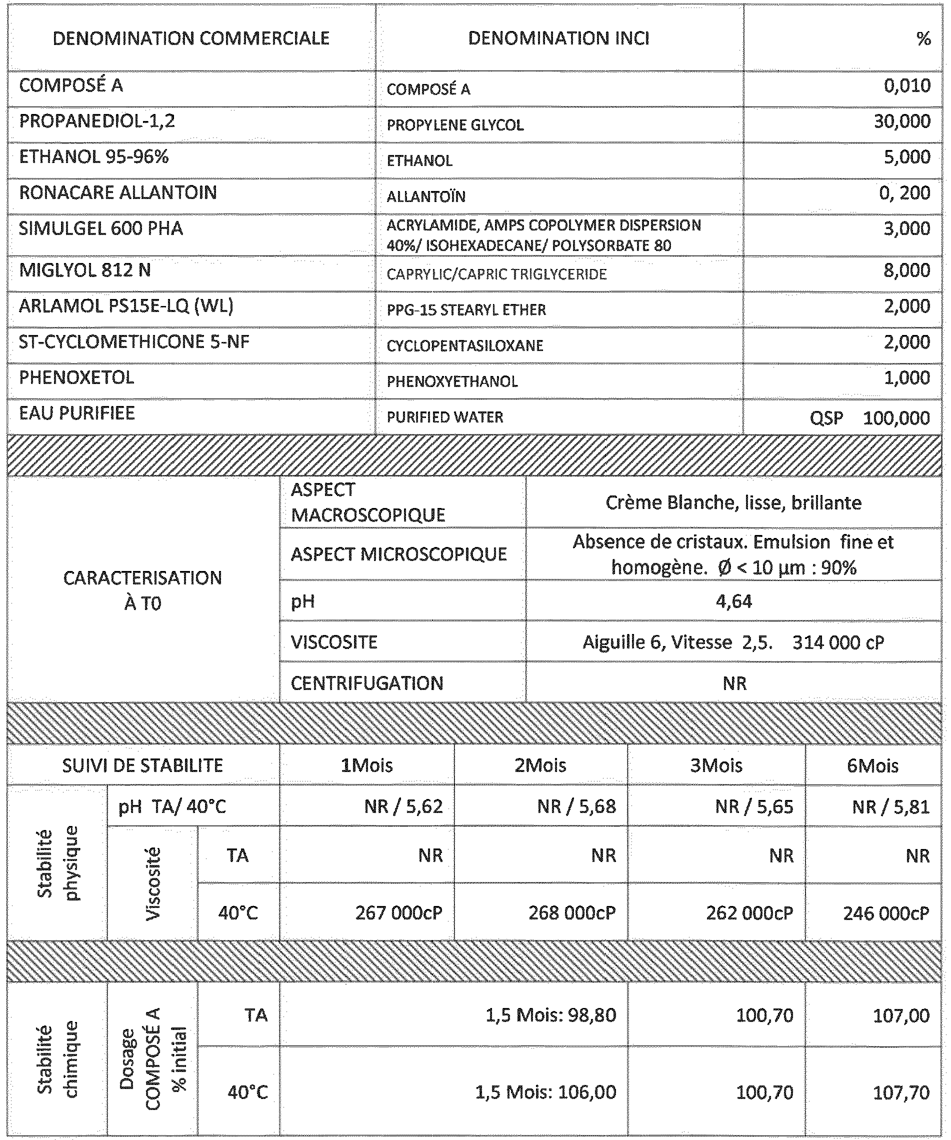

Ces résultats montrent une bonne stabilité physique et chimique de l'actif et de la composition dans son ensemble dans le temps.

### Formule 7

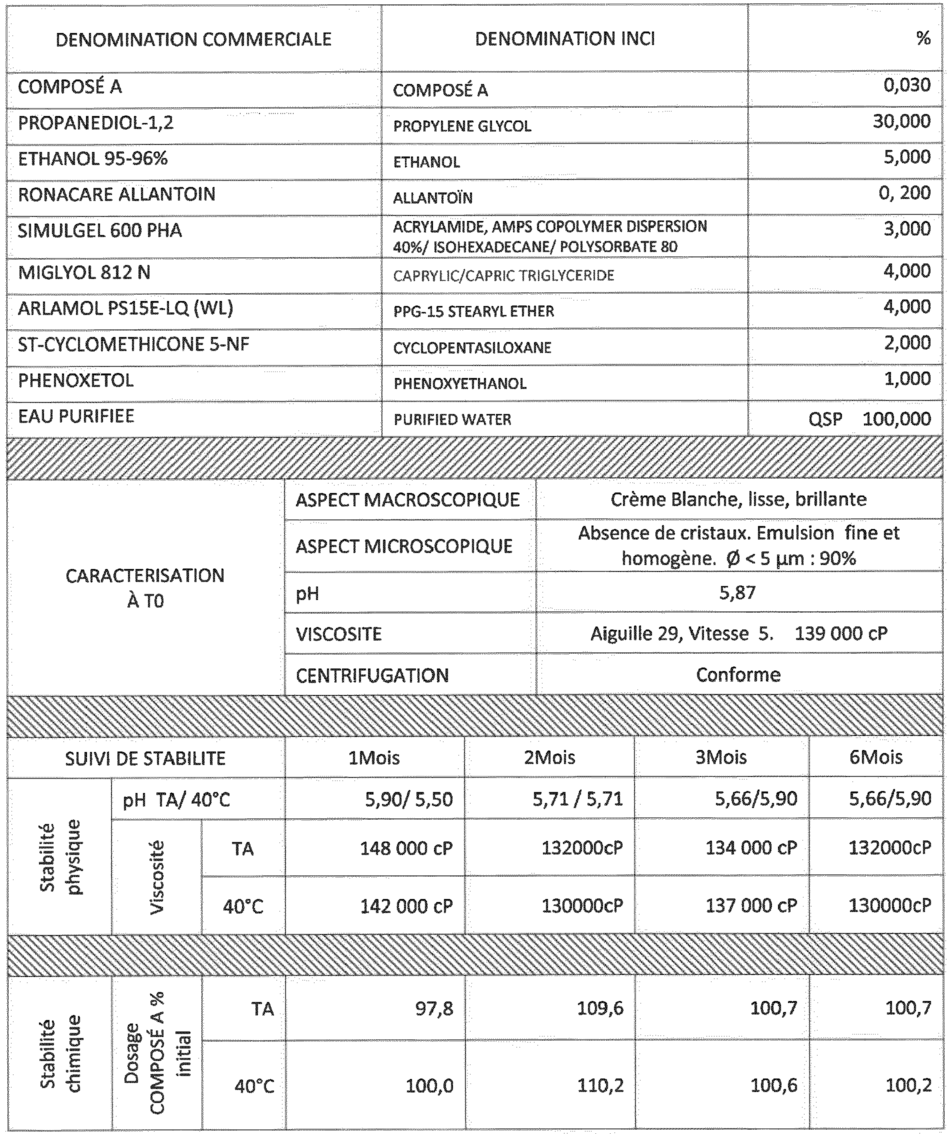

Ces résultats montrent une bonne stabilité physique et chimique de l'actif et de la composition dans son ensemble dans le temps.

### Formule 8

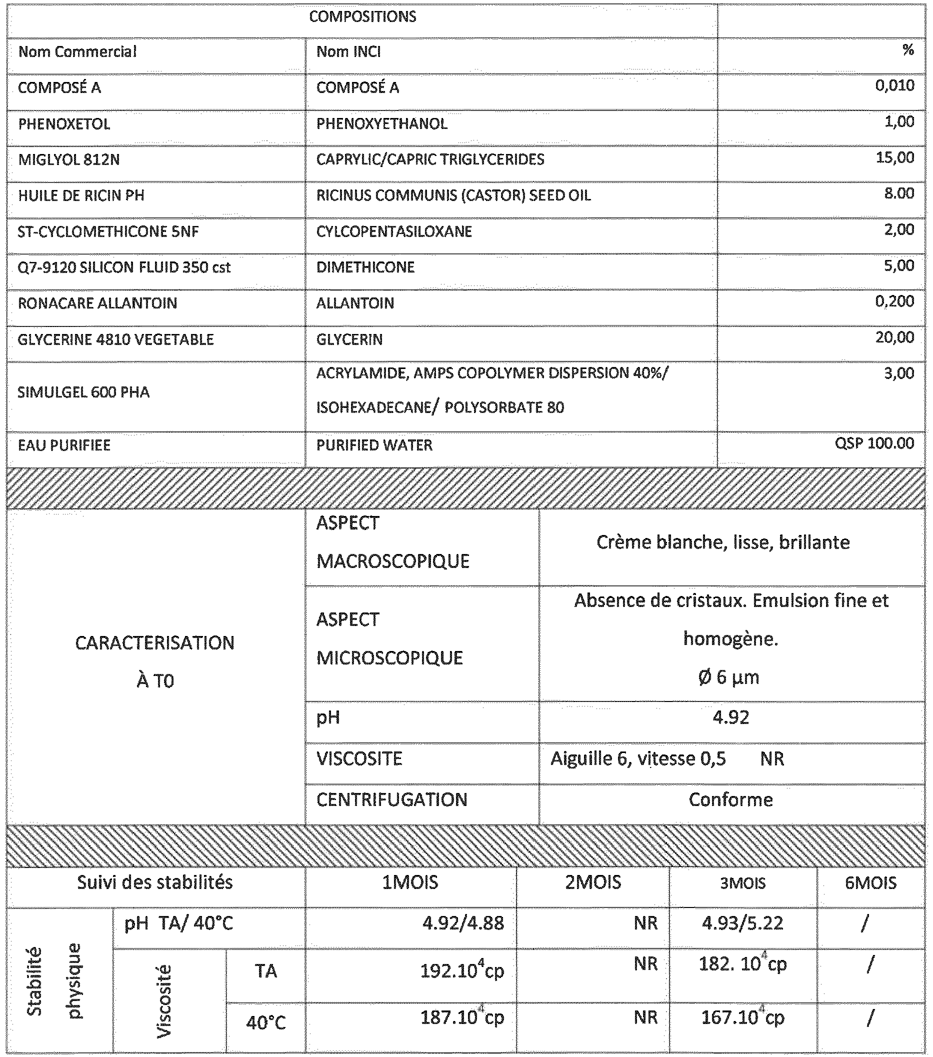

Ces résultats montrent une bonne stabilité physique de la composition dans son ensemble dans le temps.

### Formule 9

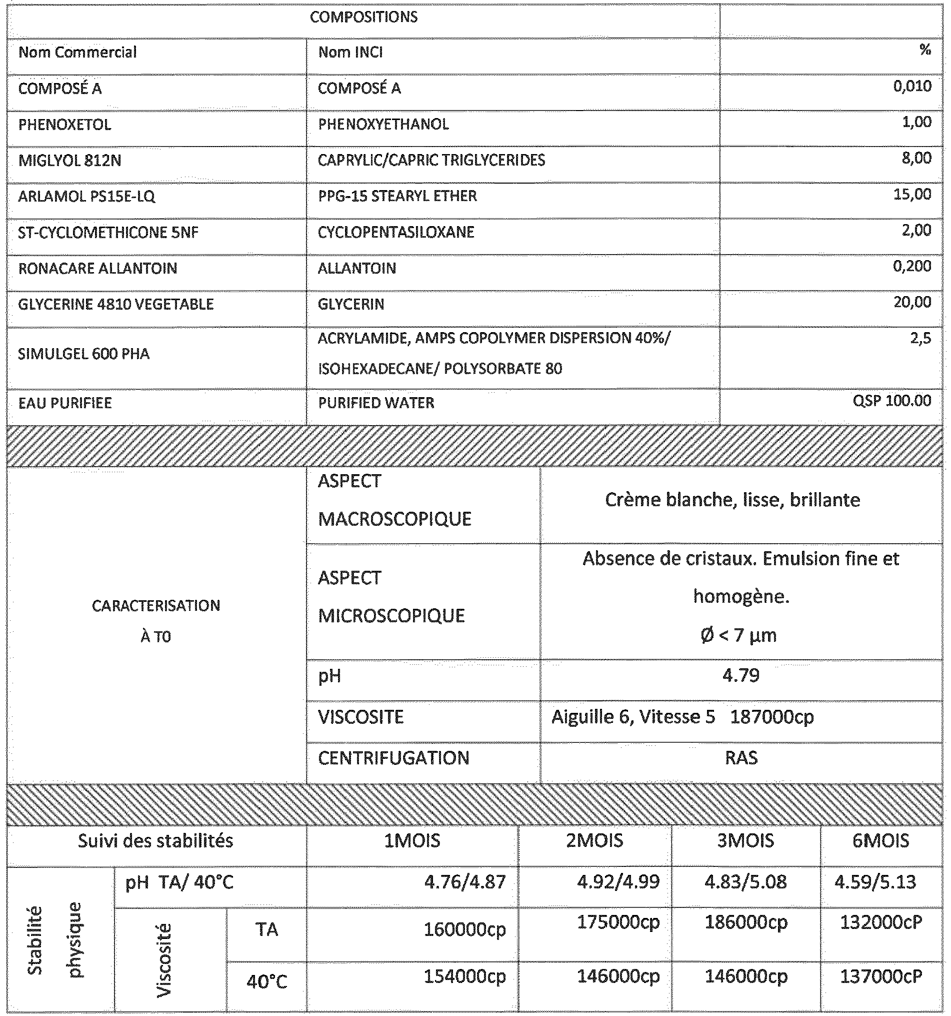

Ces résultats montrent une bonne stabilité physique de la composition dans son ensemble dans le temps.

### Formule 10

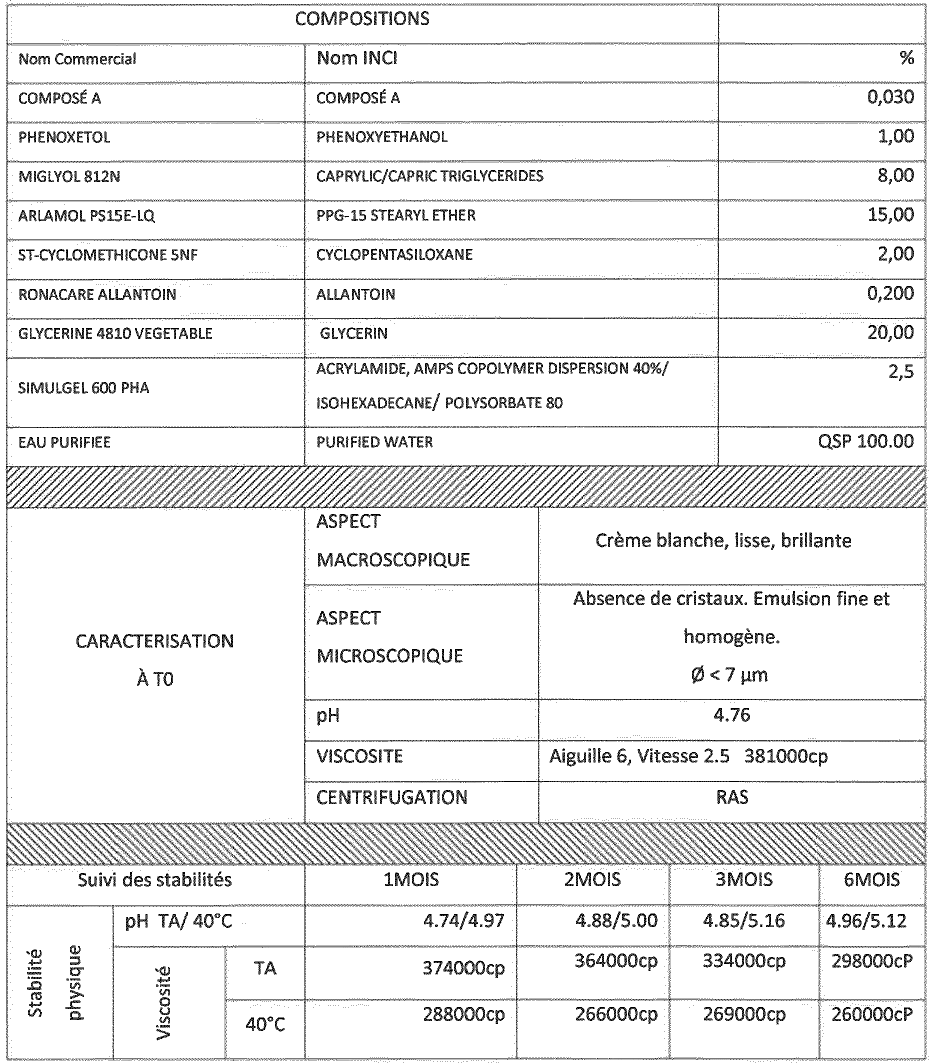

Ces résultats montrent une bonne stabilité physique de la composition dans son ensemble dans le temps.

### Formule 11

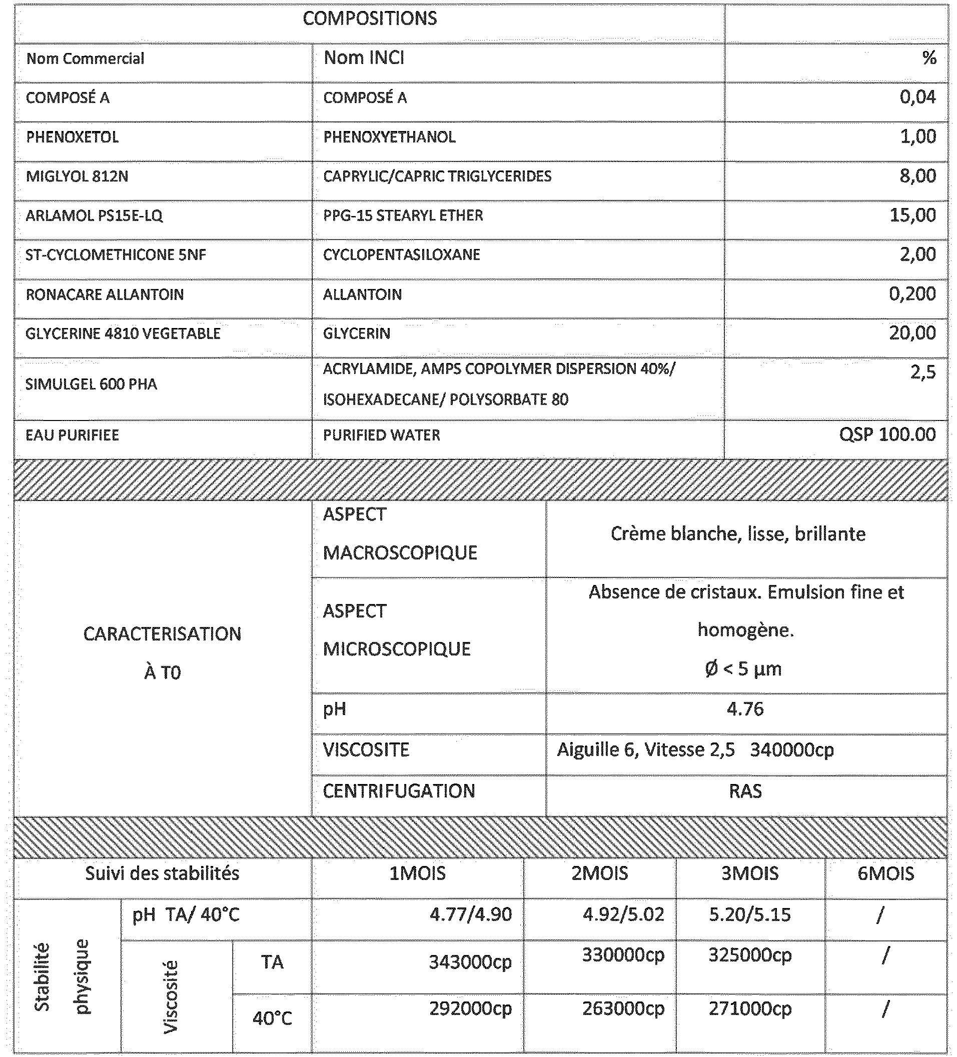

Ces résultats montrent une bonne stabilité physique de la composition dans son ensemble dans le temps.

### Formule 12

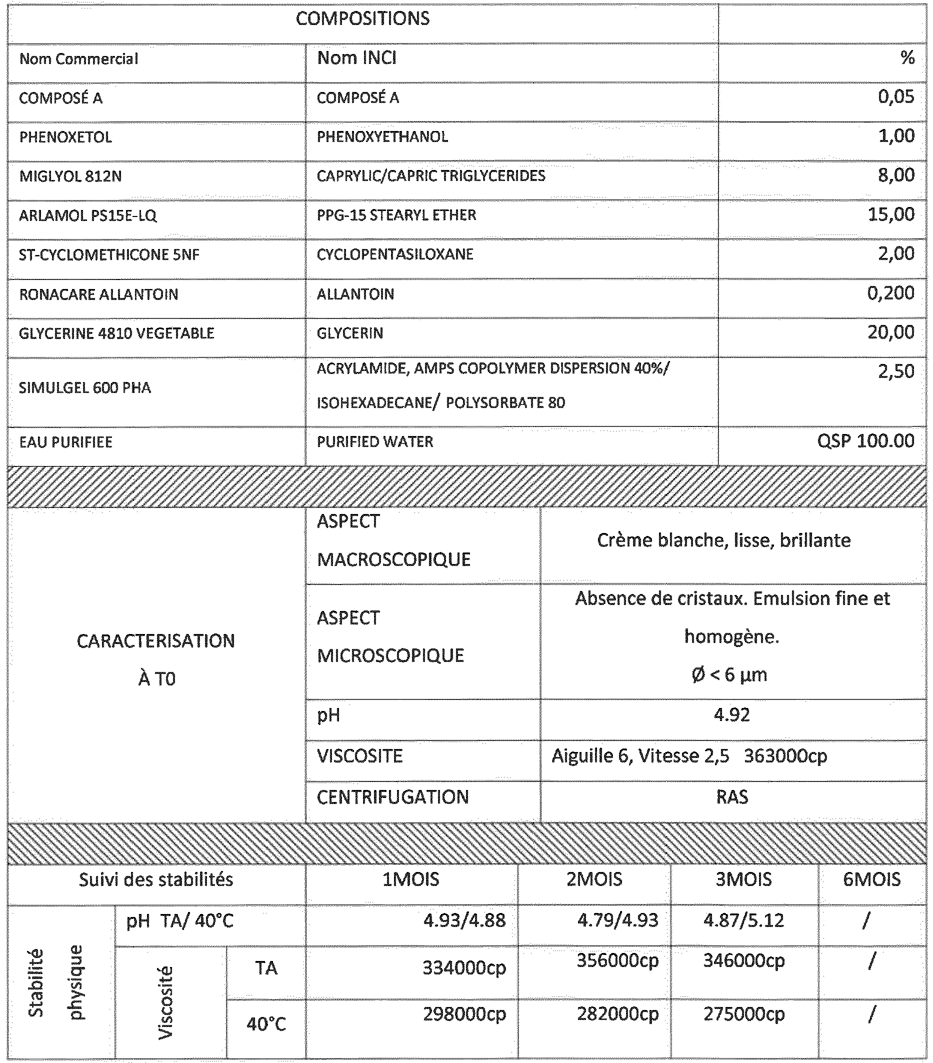

Ces résultats montrent une bonne stabilité physique de la composition dans son ensemble dans le temps.

### Formule 13

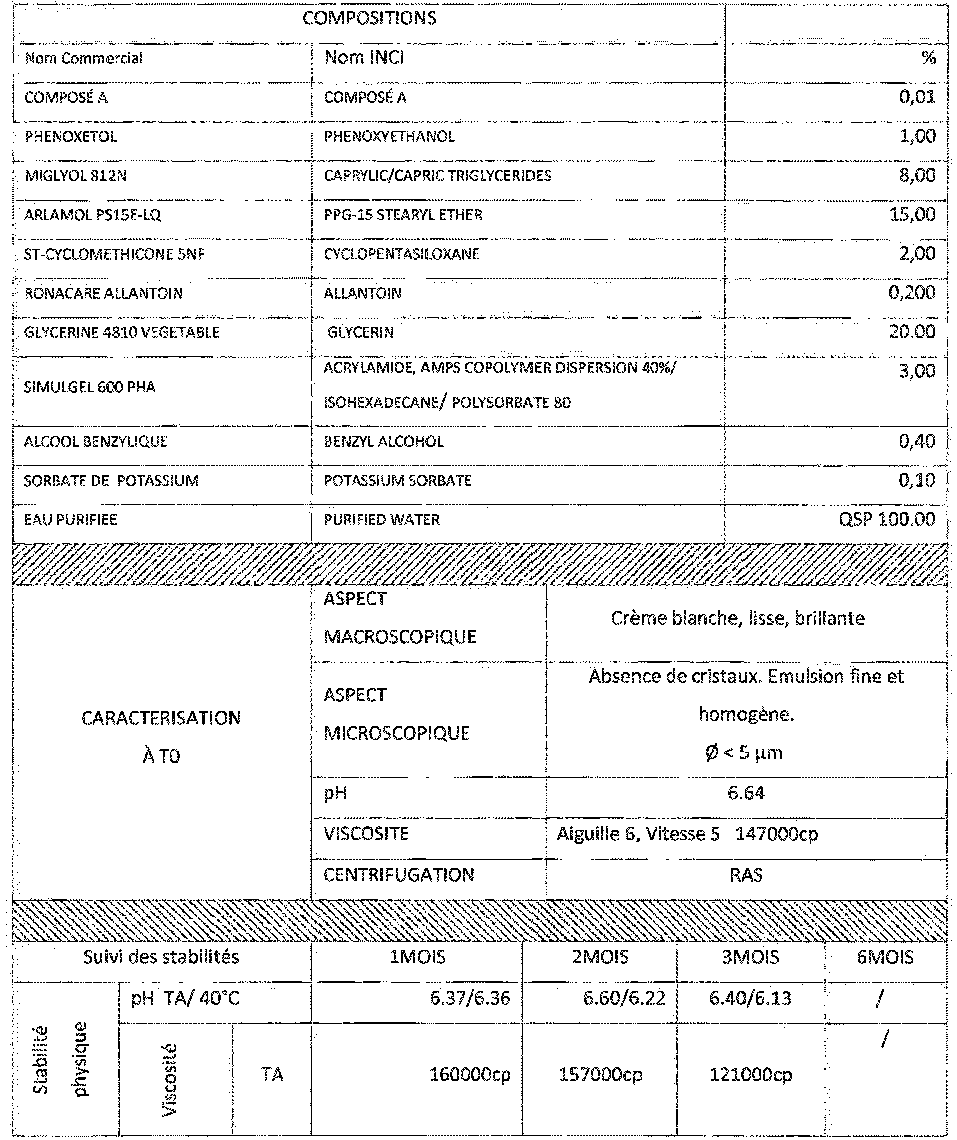

Ces résultats montrent une bonne stabilité physique de la composition dans son ensemble dans le temps.

### Formule 14

| COMPOSITIONS | | | | | |
|---|---|---|---|---|---|
| Nom Commercial | | Nom INCI | | % | |
| COMPOSÉ A | | COMPOSÉ A | | 0,01 | |
| PHENOXETOL | | PHENOXYETHANOL | | 1,00 | |
| MIGLYOL 812N | | CAPRYLIC/CAPRIC TRIGLYCERIDES | | 8,00 | |
| ARLAMOL PS15E-LQ | | PPG-15 STEARYL ETHER | | 15,00 | |
| ST-CYCLOMETHICONE 5NF | | CYCLOPENTASILOXANE | | 2,00 | |
| RONACARE ALLANTOIN | | ALLANTOIN | | 0,200 | |
| GLYCERINE 4810 VEGETABLE | | GLYCERIN | | 20.00 | |
| SIMULGEL 600 PHA | | ACRYLAMIDE,AMPS COPOLYMER DISPERSION 40%/ ISOHEXADECANE/ POLYSORBATE 80 | | 3,00 | |
| BENZOATE DE SODIUM | | SODIUM BENZOATE | | 0,20 | |
| GLUCONO-DELTA-LACTONE SG | | GLUCONOLACTONE | | 0,25 | |
| EAU PURIFIEE | | PURIFIED WATER | | QSP 100.00 | |
| | | | | | |
| CARACTERISATION À T0 | | ASPECT MACROSCOPIQUE | Crème blanche, lisse, brillante | | |
| | | ASPECT MICROSCOPIQUE | Absence de cristaux. Emulsion fine et homogène. Ø < 8 µm | | |
| | | pH | 5.44 | | |
| | | VISCOSITE | Aiguille 6, Vitesse 20 36450cp | | |
| | | CENTRIFUGATION | RAS | | |
| | | | | | |
| Suivi des stabilités | | 1MOIS | 2MOIS | 3MOIS | 6MOIS |
| Stabilité physique | pH TA/40°C | 4.59/4.58 | 4.70/4.78 | NR | NR |

Ces résultats montrent une bonne stabilité physique de la composition dans son ensemble dans le temps.

### Formule 15

| COMPOSITIONS | | | |
|---|---|---|---|
| Nom Commercial | Nom INCI | % | |
| COMPOSÉ A | COMPOSÉ A | 0,01 | |
| PHENOXETOL | PHENOXYETHANOL | 1,00 | |
| MIGLYOL 812N | CAPRYLIC/CAPRIC TRIGLYCERIDES | 8,00 | |
| ARLAMOL PS15E-LQ | PPG-15 STEARYL ETHER | 15,00 | |

| COMPOSITIONS | | | |
|---|---|---|---|
| Nom Commercial | Nom INCI | | % |
| ST-CYCLOMETHICONE 5NF | CYCLOPENTASILOXANE | | 2,00 |
| RONACARE ALLANTOIN | ALLANTOIN | | 0,200 |
| GLYCERINE 4810 VEGETABLE | GLYCERIN | | 20.00 |
| SIMULGEL 600 PHA | ACRYLAMIDE,AMPS COPOLYMER DISPERSION 40%/ ISOHEXADECANE/ POLYSORBATE 80 | | 3,00 |
| ALCOOL BENZYLIQUE | BENZYL ALCOHOL | | 0,80 |
| SORBATE DE POTASSIUM | POTASSIUM SORBATE | | 0,20 |
| TITRIPLEX III | DISODIUM EDTA | | 0,10 |
| EAU PURIFIEE | PURIFIED WATER | | QSP 100.00 |
| | | | |
| CARACTERISATION À T0 | ASPECT MACROSCOPIQUE | Crème blanche, lisse, brillante | |
| | ASPECT MICROSCOPIQUE | Absence de cristaux. Emulsion fine et homogène. Ø < 6 µm | |
| | pH | 6.34 | |
| | VISCOSITE | Aiguille 5, Vitesse 5 67920cp | |
| | CENTRIFUGATION | RAS | |

### Formule 16

| COMPOSITIONS | | | |
|---|---|---|---|
| Nom Commercial | Nom INCI | % | |
| COMPOSÉ A | COMPOSÉ A | 0,01 | |
| PHENOXETOL | PHENOXYETHANOL | 1,00 | |
| MIGLYOL 812N | CAPRYLIC/CAPRIC TRIGLYCERIDES | 8,00 | |
| ARLAMOL PS15E-LQ | PPG-15 STEARYL ETHER | 15,00 | |
| ST-CYCLOMETHICONE 5NF | CYCLOPENT ASILOXANE | 2,00 | |
| RONACARE ALLANTOIN | ALLANTOIN | 0,200 | |
| GLYCERINE 4810 VEGETABLE | GLYCERIN | 20.00 | |
| SIMULGEL 600 PHA | ACRYLAMIDE,AMPS COPOLYMER DISPERSION 40%/ ISOHEXADECANE/ POLYSORBATE 80 | 3,00 | |
| ACIDE BENZOIQUE | BENZOIC ACID | 0,20 | |
| SORBATE DE POTASSIUM | POTASSIUM SORBATE | 0,20 | |
| TITRIPLEX III | DISODIUM EDTA | 0,10 | |

| COMPOSITIONS | | | |
|---|---|---|---|
| Nom Commercial | Nom INCI | | % |
| EAU PURIFIEE | PURIFIED WATER | | QSP 100.00 |
| | | | |
| CARACTERISATION À T0 | ASPECT MACROSCOPIQUE | Crème blanche, lisse, brillante | |
| | ASPECT MICROSCOPIQUE | Absence de cristaux. Emulsion fine et homogène. Ø < 10µm | |
| | pH | 5.43 | |
| | VISCOSITE | Aiguille 5, Vitesse 5 59520cp | |
| | CENTRIFUGATION | RAS | |

### Formule 17

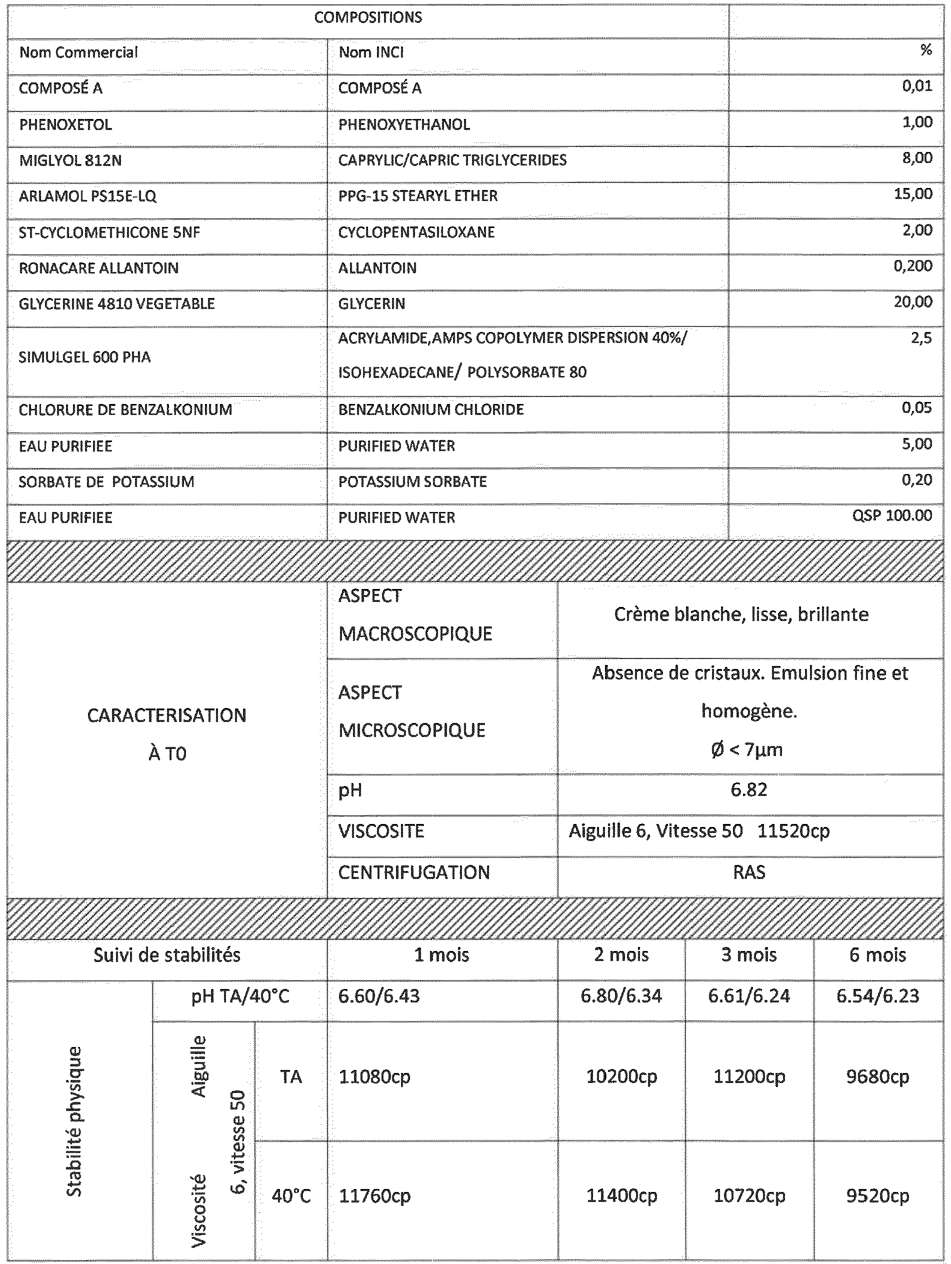

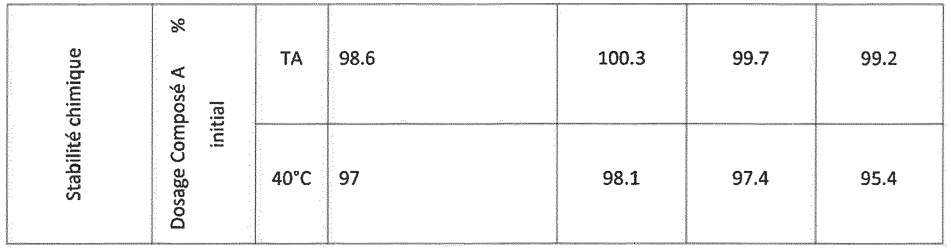

Ces résultats montrent une bonne stabilité physique et chimique de l'actif et de la composition dans son ensemble dans le temps.

### Formule 18

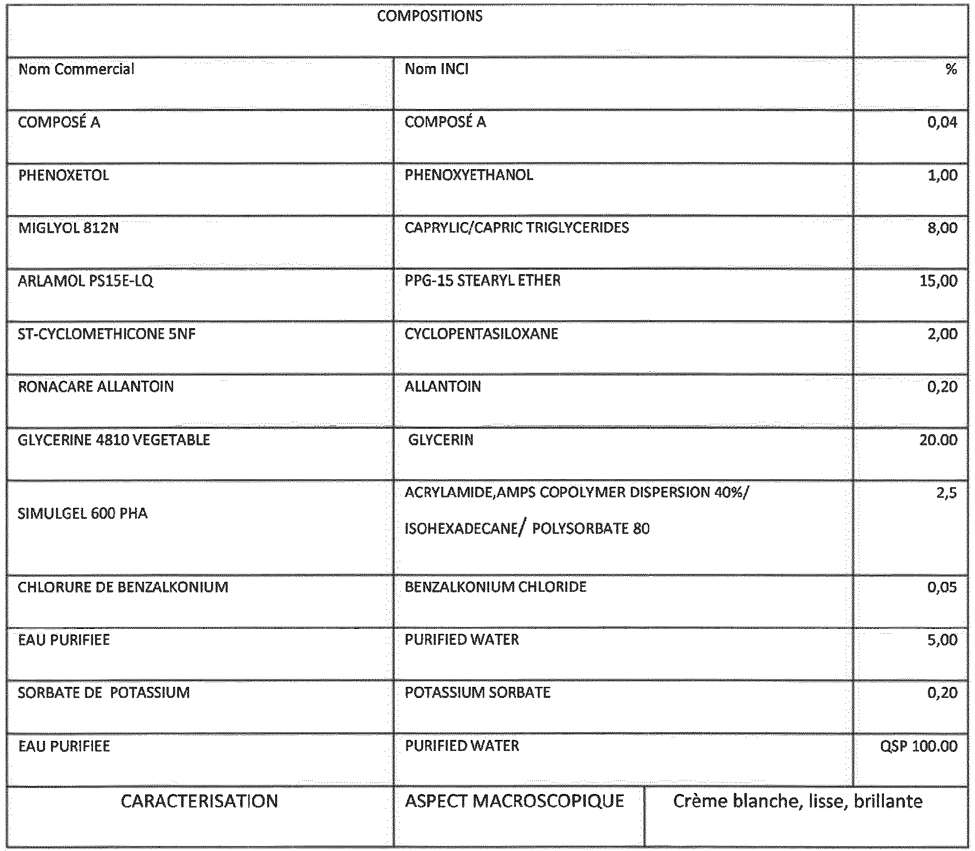

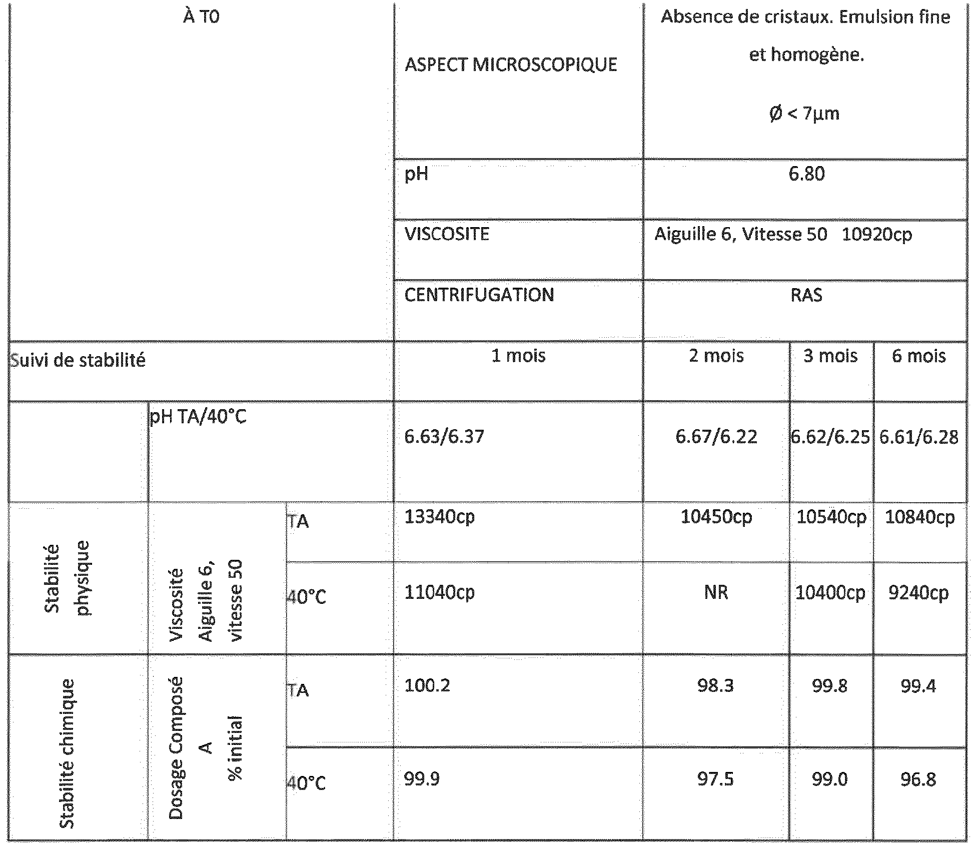

Ces résultats montrent une bonne stabilité physique et chimique de l'actif et de la composition dans son ensemble dans le temps.

### Formule 19 :

| COMPOSITIONS | | | |
|---|---|---|---|
| Nom Commercial | Nom INCI | | % |
| COMPOSÉ A | COMPOSÉ A | | 0,01 |
| PHENOXETOL | PHENOXYETHANOL | | 1,00 |
| MIGLYOL 812N | CAPRYLIC/CAPRIC TRIGLYCERIDES | | 8,00 |
| ARLAMOL PS15E-LQ | PPG-15 STEARYL ETHER | | 15,00 |
| ST-CYCLOMETHICONE 5NF | CYCLOPENT ASI LOXANE | | 2,00 |
| RONACARE ALLANTOIN | ALLANTOIN | | 0,20 |
| GLYCERINE 4810 VEGETABLE | GLYCERIN | | 10.00 |

| Nom Commercial | Nom INCI | | % |
|---|---|---|---|
| SIMULGEL 600 PHA | ACRYLAMIDE,AMPS COPOLYMER DISPERSION 40%/ ISOHEXADECANE/ POLYSORBATE 80 | | 3,00 |
| GLUCONO-DELTA-LACTONE SG | GLUCONOLACTONE | | 0,25 |
| PROBENZ SP | SODIUM BENZOATE | | 0,20 |
| PROPYLENZ GLYCOL | PROPYLENE GLYCOL | | 10,00 |
| EAU PURIFIEE | PURIFIED WATER | | QSP 100.00 |
| CARACTERISATION À T0 | ASPECT MACROSCOPIQUE | Crème blanche, lisse, brillante | |
| | ASPECT MICROSCOPIQUE | Absence de cristaux. Emulsion fine et homogène. | |
| | pH | 5.20 | |
| | VISCOSITE | Aiguille 6, Vitesse 10 59200cp | |
| | CENTRIFUGATION | RAS | |

### Formule 20 :

| COMPOSITIONS | | | |
|---|---|---|---|
| Nom Commercial | Nom INCI | | % |
| COMPOSÉ A | COMPOSÉ A | | 0,01 |
| PHENOXETOL | PHENOXYETHANOL | | 1,00 |
| MIGLYOL 812N | CAPRYLIC/CAPRIC TRIGLYCERIDES | | 8,00 |
| ARLAMOL PS15E-LQ | PPG-15 STEARYL ETHER | | 15,00 |
| ST-CYCLOMETHICONE 5NF | CYCLOPENT ASILOXANE | | 2,00 |
| RONACARE ALLANTOIN | ALLANTOIN | | 0,20 |
| GLYCERINE 4810 VEGETABLE | GLYCERIN | | 13,00 |
| SIMULGEL 600 PHA | ACRYLAMIDE,AMPS COPOLYMER DISPERSION 40%/ ISOHEXADECANE/ POLYSORBATE 80 | | 3,00 |
| GLUCONO-DELTA-LACTONE SG | GLUCONOLACTONE | | 0,25 |
| ALCOOL BENZYLIQUE | BENZYL ALCOHOL | | 1,00 |
| PROBENZ SP | SODIUM BENZOATE | | 0,20 |
| PROPYLENE GLYCOL | PROPYLENE GLYCOL | | 7,00 |
| EAU PURIFIEE | PURIFIED WATER | | QSP 100.00 |

| Nom Commercial | Nom INCI | | % |
|---|---|---|---|
| CARACTERISATION À T0 | ASPECT MACROSCOPIQUE | Crème blanche, lisse, brillante | |
| | ASPECT MICROSCOPIQUE | Absence de cristaux. Emulsion fine et homogène. | |
| | pH | 4.89 | |
| | VISCOSITE | Aiguille 29, Vitesse 20 29650cp | |
| | CENTRIFUGATION | RAS | |

### Formule 21 :

| COMPOSITIONS | | | |
|---|---|---|---|
| Nom Commercial | Nom INCI | | % |
| COMPOSÉ A | COMPOSÉ A | | 0,01 |
| PHENOXETOL | PHENOXYETHANOL | | 1,00 |
| MIGLYOL 812N | CAPRYLIC/CAPRIC TRIGLYCERIDES | | 8,00 |
| ARLAMOL PS15E-LQ | PPG-15 STEARYL ETHER | | 15,00 |
| ST-CYCLOMETHICONE 5NF | CYCLOPENTASILOXANE | | 2,00 |
| RONACARE ALLANTOIN | ALLANTOIN | | 0,20 |
| GLYCERINE 4810 VEGETABLE | GLYCERIN | | 15,00 |
| SIMULGEL 600 PHA | ACRYLAMIDE, AMPS COPOLYMER DISPERSION 40%/ ISOHEXADECANE/ POLYSORBATE 80 | | 3,00 |
| GLUCONO-DELTA-LACTONE SG | GLUCONOLACTONE | | 0,25 |
| ALCOOL BENZYLIQUE | BENZYL ALCOHOL | | 1,00 |
| PROBENZ SP | SODIUM BENZOATE | | 0,20 |
| PROPYLENE GLYCOL | PROPYLENE GLYCOL | | 5,00 |
| EAU PURIFIEE | PURIFIED WATER | | QSP 100.00 |
| CARACTERISATION À T0 | ASPECT MACROSCOPIQUE | Crème blanche, lisse, brillante | |
| | ASPECT MICROSCOPIQUE | Absence de cristaux. Emulsion fine et homogène. | |
| | pH | 4.82 | |
| | VISCOSITE | Aiguille 29, Vitesse 12 48583cp | |
| | CENTRIFUGATION | RAS | |

### Formule 22 :

| COMPOSITIONS | | | |
|---|---|---|---|
| Nom Commercial | Nom INCI | | % |
| COMPOSÉ A | COMPOSÉ A | | 0,01 |
| PHENOXETOL | PHENOXYETHANOL | | 1,00 |
| MIGLYOL 812N | CAPRYLIC/CAPRIC TRIGLYCERIDES | | 8,00 |
| ARLAMOL PS15E-LQ | PPG-15 STEARYL ETHER | | 15,00 |
| ST-CYCLOMETHICONE 5NF | CYCLOPENT ASILOXANE | | 2,00 |
| RONACARE ALLANTOIN | ALLANTOIN | | 0,20 |
| TITRIPLEX III | EDTA | | 0,20 |
| GLYCERINE 4810 VEGETABLE | GLYCERIN | | 20,00 |
| SIMULGEL 600 PHA | ACRYLAMIDE, AMPS COPOLYMER DISPERSION 40%/ ISOHEXADECANE/ POLYSORBATE 80 | | ' 2,50 |
| GLUCONO-DELTA-LACTONE SG | GLUCONOLACTONE | | 0,25 |
| ALCOOL BENZYLIQUE | BENZYL ALCOHOL | | 1,00 |
| PROBENZ SP | SODIUM BENZOATE | | 0,20 |
| EAU PURIFIEE | PURIFIED WATER | | QSP 100.00 |
| CARACTERISATION À T0 | ASPECT MACROSCOPIQUE | Crème blanche, lisse, brillante | |
| | ASPECT MICROSCOPIQUE | Absence de cristaux. Emulsion fine et homogène. | |
| | pH | 4.97 | |
| | VISCOSITE | Aiguille 29, Vitesse 20 29500cp | |
| | CENTRIFUGATION | RAS | |

### Formule 23 :

| COMPOSITIONS | | | | | |
|---|---|---|---|---|---|
| Nom Commercial | | Nom INCI | | | % |
| COMPOSÉ A | | COMPOSÉ A | | | 0,06 |
| PHENOXETOL | | PHENOXYETHANOL | | | 1,00 |
| MIGLYOL 812N | | CAPRYLIC/CAPRIC TRIGLYCERIDES | | | 8,00 |
| ARLAMOL PS15E-LQ | | PPG-15 STEARYL ETHER | | | 15,00 |
| ST-CYCLOMETHICONE 5NF | | CYCLOPENTASILOXANE | | | 2,00 |
| RONACARE ALLANTOIN | | ALLANTOIN | | | 0,20 |
| GLYCERINE 4810 VEGETABLE | | GLYCERIN | | | 20,00 |
| SIMULGEL 600 PHA | | ACRYLAMIDE, AMPS COPOLYMER DISPERSION 40%/ ISOHEXADECANE/ POLYSORBATE 80 | | | 2,50 |
| CHLORURE DE BENZALKONIUM | | BENZALKONIUM CHLORIDE | | | 0,05 |

| COMPOSITIONS | | | | | |
|---|---|---|---|---|---|
| Nom Commercial | | Nom INCI | | % | |
| SORBATE DE POTASSIUM | | POTASSIUM SORBATE | | 0,20 | |
| EAU PURIFIEE | | PURIFIED WATER | | QSP 100.00 | |
| CARACTERISATION À T0 | | ASPECT MACROSCOPIQUE | Crème blanche, lisse, brillante | | |
| | | ASPECT MICROSCOPIQUE | Absence de cristaux. Emulsion fine et homogène. | | |
| | | pH | 6.64 | | |
| | | VISCOSITE | Aiguille 5, Vitesse 10 32320cp | | |
| | | CENTRIFUGATION | RAS | | |
| Suivi des stabilités | | 1MOIS | 2MOIS | 3MOIS | 6MOIS |
| Stabilité physique | pH TA/ 40°C | 6.58/6.41 | 6.44/6.23 | NR | 6.48/6.17 |
| | Viscosité TA Aiguille 5 Vitesse 1 | 309000cp | 251000cp | 287000cp | 300000cp |
| | Viscosité 40°C Aiguille 5 Vitesse 1 | 320000cp | 374000cp | 324000cp | 317000cp |

Ces résultats montrent une bonne stabilité physique de la composition dans son ensemble dans le temps.

### Exemple 3 : Caractérisation des formulations par des études de pénétration cutanée sur peau humaine:

Les études de pénétration cutanée permettent de caractériser les formulations, et de mettre en évidence des paramètres propres à chacune des formulations.

Deux types d'études de pénétration cutanée sur peau humaine ex vivo ont été réalisés. Dans ces études le composé A correspond à l'acide 3"-tert-butyl-4'-(2-hydroxy-ethoxy)-4"-pyrrolidin-1-yl-[1,1';3',1"]-terphenyl-4-carboxylique.

Le gel de référence étant décrit comme suivant :

| Constituants | % en poids |
|---|---|
| Composé A | 0.01 |
| Propylene Glycol | 30.00 |
| Ethanol 95-96% | 67.99 |
| Klucel HF Pharma | 2.00 |

### 1- Etude de pénétration cutanée « temps unique »:

Dans cette étude, la formule est appliquée pendant 16h à la surface de la peau. A la fin de l'application, le composé A (COMPOSÉ A) est quantifié dans les différents compartiments de la peau : stratum corneum, épiderme, derme et liquide récepteur selon une méthode de bioanalyse validée.

Les détails de l'application cutanée sont donnés dans le tableau ci-dessous.

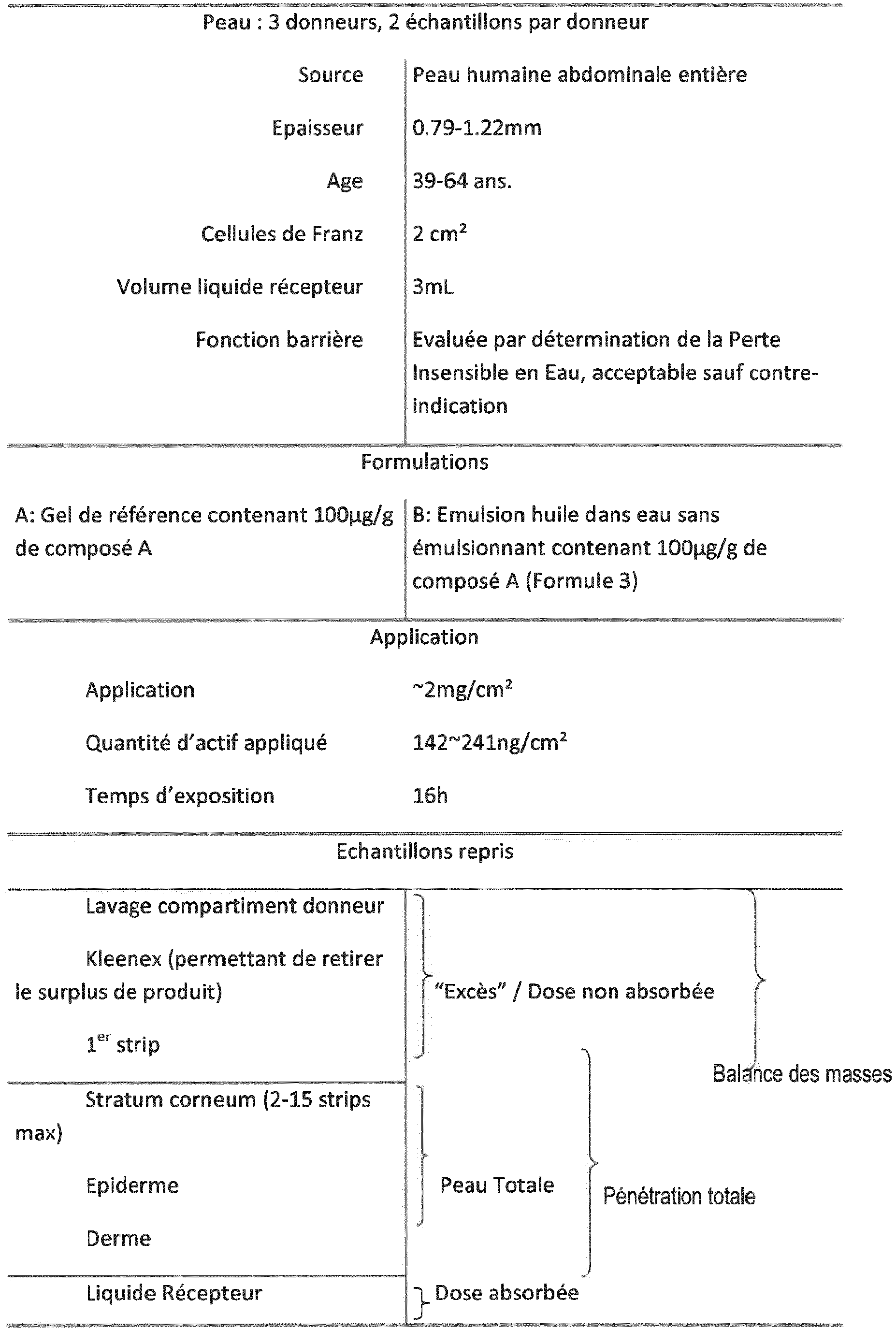

La bioanalyse a été réalisée par spectrométrie de masse en tandem par ionisation electrospray positive, et utilisant un appareil Xevo (Waters). La limite de quantification pour le composé A est de 1ng/mL.

Les conditions de LC/MS/MS mises au point ont permis de détecter jusqu'à 0,1% de la dose appliquée dans chacun des compartiments (dose non absorbée, stratum, épiderme, derme et liquide récepteur).

Les conditions techniques sont données dans le tableau ci-dessous.

| | | | | | | |
|---|---|---|---|---|---|---|
| Colonne LC | Hypersil gold 50*2.1mm (UPLC) | | | | | |
| Phase Mobile | Phase A : ACN + 0.1% Formic Acid | | | | | |
| | Phase B : H₂O + 0.1% Formic acid | | | | | |
| Lavage aiguille | ACN | | | | | |
| Lavage septum | ACN/H₂O 50:50 | | | | | |
| Gradient | Temps (min) | débit | %A | %B | Courbe | |
| | 1. Initial | 0.700 | 15.0 | 85.0 | 0 | |
| | 2.2.5 | 0.700 | 90.0 | 10.0 | 6 | |
| | 3.3.20 | 0.700 | 90.0 | 10.0 | 6 | |
| | 4.3.25 | 0.700 | 15.0 | 85.0 | 6 | |
| Température de colonnes | | | | | | |
| Detection MSMS | ESI+ MRM (Electrospray Positif) | | | | | |
| | Canal Réaction | Dwell (secs) | Voltage (cône) | Col. Energy | Tr (min) | Composé |
| | 1: 460.26 > 318.20 | 0.100 | 50.0 | 40.0 | 1.58 | Composé A |
| | 1: 464.06 > 372.10 | 0.100 | 55.0 | 40.0 | 1.58 | Standard Into |
| Volume Injection | 5µL | | | | | |
| Temps de run | 4 minutes | | | | | |

Dans ce type d'étude « point unique », les paramètres retenus sont :
a. Le profil de distribution dans les différents compartiments (données qualitatives)
b. La pénétration dans le compartiment épiderme + derme (données numériques)

### a-Profil de distribution dans les différents compartiments :

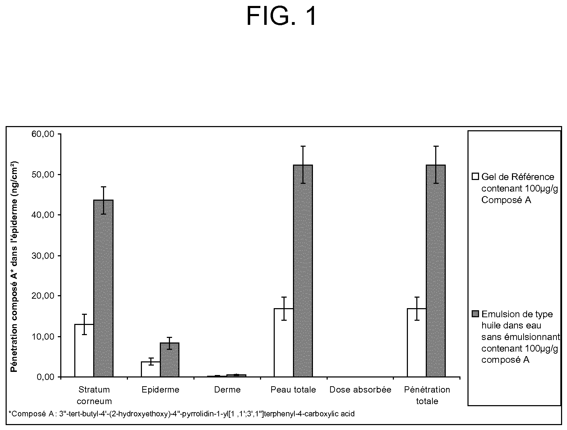

Ce profil est présenté en Figure 1.

Le profil de distribution entre les différents compartiments est du même type pour les 2 formules évaluées : accumulation dans le stratum corneum, taux de pénétration plus faible dans l'épiderme, et pénétration très faible dans le derme. Le composé A n'est pas détecté dans le liquide récepteur.

### b-Valeurs de pénétration dans le compartiment épiderme + derme :

Les valeurs de pénétration pour l'émulsion de type huile dans eau sans émulsionnant contenant 100µg/g (0.01%) de composé A sont comprises entre 6.8ng/cm² et 10.6ng/cm².

Les niveaux de pénétration du composé A après application de l'émulsion type huile dans eau sans émulsionnant tendent à être plus élevés que ceux obtenus après application du gel de référence.

### 2- Etude de cinétique de pénétration :

Dans ce type d'étude, la pénétration de l'actif est quantifiée dans chaque compartiment de la peau après 0.5h, 1h, 3h 6h et 24h d'application. Une cinétique de pénétration dans chaque compartiment est alors déterminée et caractérisée.

Les détails de l'application cutanée sont donnés dans le tableau ci-dessous :

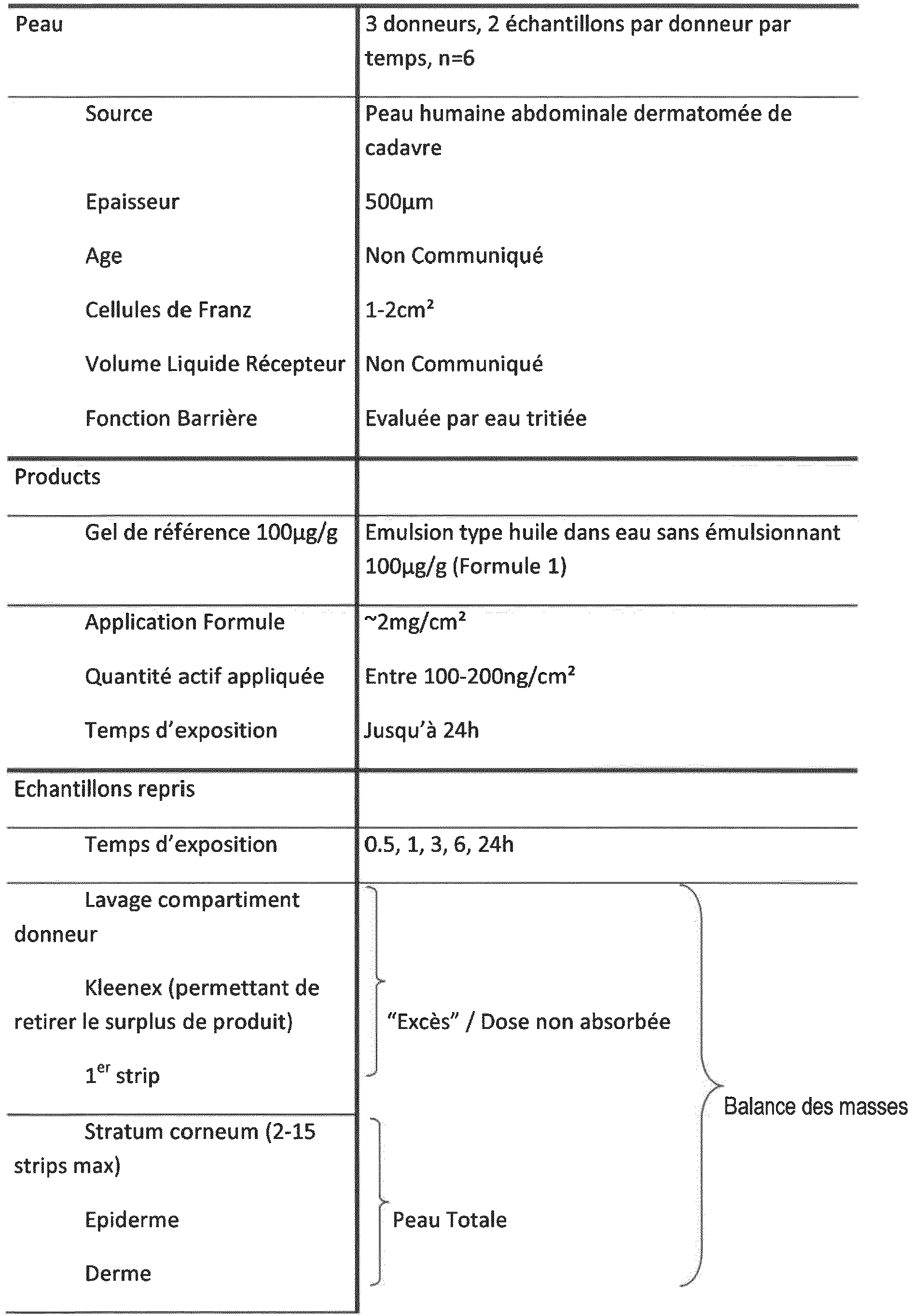

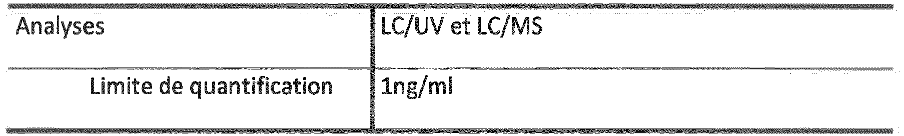

La quantité d'actif dans chaque compartiment à chaque temps a été déterminée par LC/UV ou par LC/MS. La méthode de bioanalyse a été validée de sorte à détecter au minimum 0,1% de la dose appliquée dans chaque compartiment.

Dans ce type d'étude, les paramètres retenus sont :
a. Le profil de la cinétique de pénétration dans l'épiderme (données qualitatives)
b. La vitesse initiale de la pénétration dans l'épiderme
c. La quantité maximale pénétrée dans l'épiderme

### a. Profil de la cinétique de pénétration dans l'épiderme:

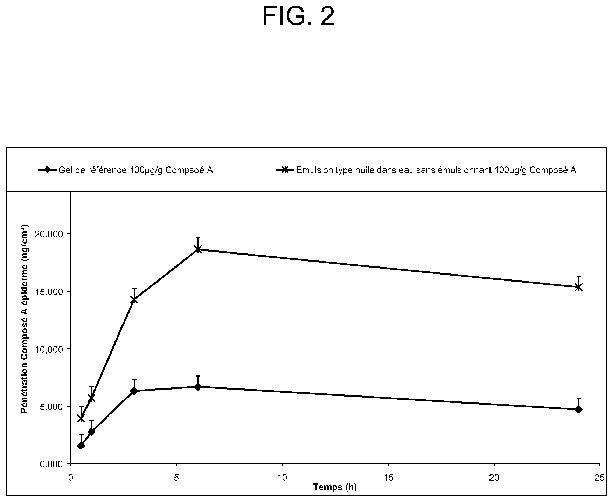

Celle-ci est présentée en figure 2.

La cinétique de libération du composé A obtenue pour l'émulsion type huile dans eau sans émulsionnant présente une pente initiale élevée, suivie d'un plafond pendant lequel la pénétration du composé A n'augmente plus au cours du temps. La formule de référence (gel) montre la même cinétique avec une libération rapide au cours des premières heures et ensuite atteinte d'un plateau.

Comme vu dans le paragraphe 1 (Etude de pénétration cutanée « temps unique »), ces deux formules ont des niveaux de pénétration à 16h qui sont différents, la pénétration du composé A dans l'épiderme après application de l'émulsion huile dans eau sans émulsionnant tend à être plus élevée que celle obtenue après application du gel de référence.

### b. Vitesse initiale de la cinétique :

La valeur de vitesse initiale de la cinétique ou pente sur les 3 premières heures est de 4.2ng/cm²/h.

### c. Quantité maximale dans l'épiderme:

La quantité maximale dans l'épiderme est de 18.7ng/cm².

### Exemple 4 Etude de tolérance

Dans cette étude :
- 10 sujets ont reçu 2 grammes de Gel (Gel de référence) appliqués sur 1000 cm² pendant 4 semaines Par gel de référence on entend un gel décrit comme suivant :

| Constituants | % |
|---|---|
| Composé A | 0.01 |
| Propylene Glycol | 30.00 |
| Ethanol 95-96% | 67.99 |
| Klucel HF Pharma | 2.00 |

- 10 sujets ont reçu 2 grammes de Crème B (émulsion huile dans eau sans émulsionnant-Formule 1) appliqués sur 1000 cm² pendant 4 semaines

Au cours de l'étude, les investigateurs avaient la possibilité de changer de zone d'application en cas d'irritation trop importante.

Les résultats de l'étude sont présentés dans le tableau ci-après : Les valeurs numériques sont le nombre de patient pour lesquels un changement de zone a été effectué (valeur N dans le tableau), la valeur entre parenthèse est le pourcentage correspondant à N.

**Evolution du pourcentage de de sujet pour lesquels il n'y a pas de changement de zone d'application**

| | | Gel 50µg/g 1000 cm² | Crème B 50µg/g 1000 cm2 | Gel 50µg/g 2000 cm² | Gel 100µg/g 1000 cm² | Gel 25µg/g 1000 cm² |
|---|---|---|---|---|---|---|
| Jour 2 | N(%) | - | - | - | - | - |
| Jour 4 | N(%) | 1(10) | - | - | - | - |
| Jour 5 | N(%) | - | 1(10) | 2(20) | 1(10) | - |
| Jour 6 | N(%) | 1(10) | - | - | 1(10) | - |
| Jour 7 | N(%) | - | - | 2(20) | 1(10) | 1(10) |
| Jour 8 | N(%) | 1(10) | 1(10) | - | 2(20) | 1(10) |
| Jour 9 | N(%) | 3(30) | 1(10) | 2(20) | 1(10) | - |
| Jour 10 | N(%) | - | - | 1(10) | 1(10) | 1(10) |
| Jour 11 | N(%) | 2(20) | 1(10) | - | 1(10) | 1(10) |
| Jour 12 | N(%) | - | 1(10) | 1(10) | - | 1(10) |
| Jour 13 | N(%) | - | 1(10) | - | - | - |
| Jour 14 | N(%) | 1(10) | 1(10) | 2(20) | 1(10) | 2(20) |
| Jour 16 | N(%) | 1(10) | 1(10) | - | 1(10) | - |
| Jour 18 | N(%) | - | - | - | - | 1(10) |
| Jour 19 | N(%) | - | - | - | - | - |
| Jour 24 | N(%) | - | 1(10) | - | - | 1(10) |
| Aucun changement au cours de l'étude | N(%) | - | 1(10) | - | - | 1(10) |

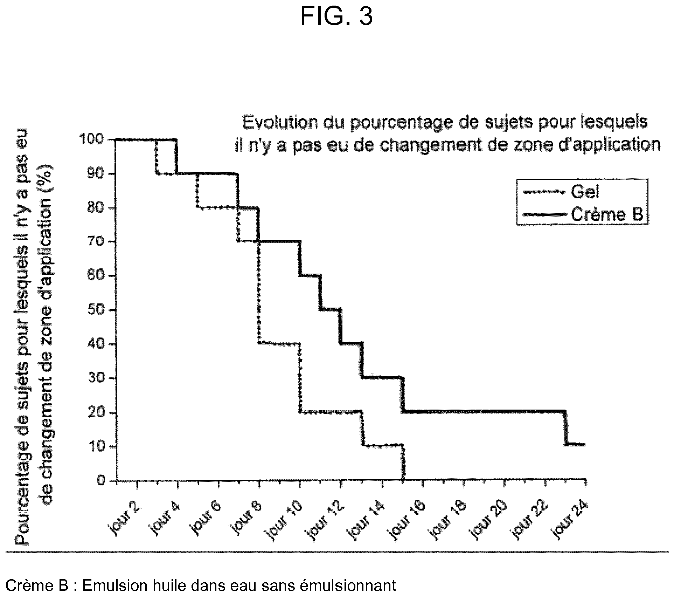

La figure 3 représente le pourcentage de sujets pour lesquels il n'y a eu aucun changement de zone d'application, en fonction de du jour d'application.

Par exemple, au jour 5, pour 90 % des sujets recevant de la Crème B, il n'y a pas eu besoin de changer de zone d'application. Autrement dit, 10 % des sujets ayant reçu de la Crème B ont montré une irritation nécessitant un changement de zone d'application.

Ainsi, on constate que l'irritation apparaît plus rapidement chez les sujets qui ont reçu le Gel que chez les sujets qui ont reçu la Crème B. C'est à partir du jour 9 que l'on observe une nette différence.

## Revendications

1. Composition de type émulsion huile dans eau comprenant
- Une phase grasse comprenant :
l'acide 3"-tert-butyl-4'-(2-hydroxy-ethoxy)-4"-pyrrolidin-1-yl-[1,1';3',1"]-terphenyl-4-carboxylique
au moins un solvant principal de l'acide 3"-tert-butyl-4'-(2-hydroxy-ethoxy)-4"-pyrrolidin-1-yl-[1,1';3',1"]-terphenyl-4-carboxylique, choisi parmi l'alcool benzylique, le laureth-4, le phénoxyéthanol, le propylène glycol monocaprylate, le pentylène glycol, le dimethyl isosorbide, et leurs mélanges, et au moins une huile co-solvante de l'acide 3"-tert-butyl-4'-(2-hydroxy-ethoxy)-4"-pyrrolidin-1-yl-[1,1';3',1"]-terphenyl-4-carboxylique, choisie parmi les triglycérides caprylique/ caprique, l'huile d'amande douce, le monocaprylate de propylène glycol, le laurate de propylène glycol, le sesquioléate de sorbitan, l'adipate de diisopropyle, l'éther de PPG-15 stéaryle, l'ester de PEG-6 et huile de noyau d'abricot, et leurs mélanges, et
- Une phase aqueuse comprenant au moins un agent gélifiant, choisi parmi les polymères d'origine végétale, les gommes, les pectines, la cellulose et ses dérivés, les polymères d'origine microbiologiques tel que la gomme xanthane, et les polymères gélifiants d'origine synthétique.

2. Composition selon la revendication 1, **caractérisée en ce que** l'agent gélifiant est choisi parmi:
- les Acrylates/C10-30 Alkyl Acrylate Crosspolymer vendu sous le nom de Pemulen TR-1 ou Pemulen TR-2
- les gélifiants de la famille des polyacrylamides comme le mélange Sodium acrylamide/acryloyldimethyltaurate copolymer / isohexadecane / polysorbate 80 vendu sous le nom de Simulgel 600PHA
- le mélange polyacrylamide/isoparaffine C13-14/laureth-7 vendu sous le nom de Sepigel 305,
- les carbomers vendus sous le nom d'Ultrez 20^{®}, d'Ultrez 10^{®}, de Carbopol 1382^{®} ou de Carbopol ETD2020NF^{®}, de Carbopol 981 ou encore Carbopol 980,
- les polysaccharides avec à titre d'exemples non limitatifs la gomme de xanthane telle que le Xantural180^{®}, la gellan gum vendu sous le nom de Kelcogel, la gomme guar, la cellulose et ses dérivés tel que la microcristalline cellulose et carboxymethyl cellulose de sodium vendue sous le nom d'Avicel CL-611, l'hydroxypropylmethylcellulose en particulier le produit vendu sous le nom de Methocel E4M premium ou l'hydroxyéthylcellulose , en particulier, le produit vendu sous le nom de Natrosol HHX 250^{®} , le sodium carboxymethylcellulose en particulier la Blanose cellulose gum 7F,
- la famille des aluminium magnésium silicates tel que le Veegum K,
- la famille des polymères acryliques couplés à des chaînes hydrophobes tel que le PEG-150/decyl/SMDI copolymer vendu sous le nom de Aculyn 44,
- la famille des amidons modifiés tels que l'amidon de pomme de terre modifié vendu sous le nom de Structure Solanace ou bien leurs mélanges,
- la famille des carraghénanes en particulier réparties sous quatre grandes familles : κ, λ, β, ω tel que les Viscarin^{®} et les Gelcarin^{®}.

3. Composition selon l'une quelconque des revendications précédentes, **caractérisée en ce que** le solvant principal est le phénoxyéthanol.

4. Composition selon l'une quelconque des revendications précédentes, **caractérisée en ce qu'**elle contient en outre un ou des additifs choisis parmi :
- un ou plusieurs agents conservateurs choisi parmi le méthyl parabène, le propyl parabène, le chlorure de benzalkonium, le phénoxyéthanol vendu sous le nom de phénoxétol, l'alcool benzylique vendu sous le nom d'alcool benzylique, le Sorbate de potassium vendu sous le nom de Sorbate de potassium, l'acide benzoïque, le 2-Bromo-2-Nitropropane-1,3-Diol vendu sous le nom de Bronopol, la Chlorhexidine, la Chlorhexidine digluconate, le chlorocrésol et ses dérivés, l'alcool éthylique et la diazolidinylurée,
- un ou plusieurs agents chélatant comme l'EDTA (ethylenediaminetetraacetic acid) et ses dérivés ou sels, la dihydroglycerine, les acides citriques et tartriques, la gluconolactone vendu sous le nom glucono-delta-lactone SG ou des mélanges de ceux-ci,
- un ou plusieurs antioxydants tel que la vitamine E et ses dérivés, comme le DL alpha Tocophérol ou l'acétate de tocophérol; la vitamine C et ses dérivés, comme l'Ascorbyl Palmitate, le Butylhydroxy toluene vendu sous le nom de Nipanox BHT,
- un ou plusieurs agents apaisants et/ou anti-irritants tels que le PPG-12/SMDI copolymer sous le nom commercial de Polyolprepolymer-2, l'acide glycyrrhetinique ou ses dérivés comme par exemple l'Enoxolone, l'acide hyaluronique tel quel ou sous sa forme hyaluronate de sodium vendu sous le nom commercial de HYAL. NA PWD PH 15-51-45, l'allantoïne vendue sous le nom de RONACARE ALLANTOINE.

5. Composition selon l'une quelconque des revendications précédentes, **caractérisée en ce qu'**elle contient en outre une huile minérale.

6. Composition selon l'une quelconque des revendications précédentes, **caractérisée en ce qu'**elle contient en outre un agent humectant.

7. Composition selon l'une quelconque des revendications précédentes, **caractérisée en ce qu'**elle contient en outre une huile silicone.

8. Composition selon l'une quelconque des revendications précédentes, **caractérisée en ce qu'**elle contient en outre de l'éthanol.

9. Composition selon l'une des revendications 1 à 8 **caractérisée en ce qu'**elle comprend les ingrédients suivants :
de 0.00001% à 1% en poids et préférentiellement de 0.0001 à 0.1 % en poids et de manière plus préférée de 0.001 à 0.1 % en poids de composé de l'acide 3"-tert-butyl-4'-(2-hydroxy-ethoxy)-4"-pyrrolidin-1-yl-[1,1';3',1"]-terphenyl-4-carboxylique
de 0,005 à 10 % en poids et préférentiellement de 1 à 5% en poids d'agent gélifiant, tel que défini dans la revendication 1,
de 0.2 à 5% en poids et préférentiellement de 0,5 à 2% en poids de solvant principal, tel que défini dans la revendication 1,
de 0.5 à 50% en poids et préférentiellement de 4 à 30% en poids d'huile(s) co-solvante(s) , tel que défini dans la revendication 1,
et, en option :
de 0 à 20% en poids et préférentiellement de 0 à 5% en poids d'huile(s) minérale(s),
de 0 à 50% en poids et préférentiellement de 5 à 35% en poids de polyol(s)
de 0 à 10% en poids et préférentiellement 0 à 4% en poids d'huile(s) de silicone
de 0 à 5% en poids et préférentiellement de 0.01 à 2% en poids d'agent(s) conservateur(s)
de 0 à 30% en poids et préférentiellement de 0 à 10% en poids d'éthanol
de 0 à 15% en poids et préférentiellement de 0.1 à 10% en poids d'additif(s).

10. Composition selon l'une quelconque des revendications précédentes, **caractérisée en ce que** la quantité maximale de principe actif absorbée dans le derme et l'épiderme 16 heures après application soit comprise entre 6 ng/cm² et 19 ng/cm².

11. Composition selon la revendication 10 **caractérisée en ce que** la quantité maximale de principe actif absorbée dans le derme et l'épiderme 16 heures après application soit comprise entre 6,8 ng/cm² et 10,6 ng/cm².

12. Composition selon l'une quelconque des revendications précédentes **caractérisée en ce que** la quantité maximale de principe actif absorbée dans l'épiderme est obtenue entre 3 et 10 heures après application.

13. Composition selon l'une des revendications 1 à 12 pour son utilisation à titre de médicament.

14. Composition selon l'une des revendications 1 à 13 pour son utilisation pour le traitement d'une ou plusieurs des pathologies suivantes :
- Les affections dermatologiques liées à un désordre de la kératinisation portant sur la différenciation et sur la prolifération cellulaire notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle ;
- Les troubles de la kératinisation, notamment les ichtyoses, les états ichtyosiformes, l'ichtyose lamellaire, la maladie de Darrier, les kératodermies palmoplantaires, les leucoplasies, le pityriasis rubra pilaire et les états leucoplasiformes, le lichen cutané ou muqueux (buccal) ;
- Les affections dermatologiques avec une composante immuno-allergique inflammatoire, avec ou sans trouble de la prolifération cellulaire, et notamment toutes les formes de psoriasis, qu'il soit cutané, muqueux ou unguéal, et même le rhumatisme psoriasique, ou encore la dermatite atopique et les différentes formes d'eczémas;
- Les désordres cutanés dus à une exposition aux rayonnements U.V. ainsi que pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique, telle la xérose, les pigmentations et les rides ;
- Toute condition liée à des proliférations dermiques ou épidermiques bénignes, qu'elles soient ou non d'origine virale telles que verrues vulgaires, les verrues planes , le molluscum contagiosum et l'épidermodysplasie verruciforme, les papillomatoses orales ou florides,;
- Les désordres dermatologiques tels que les dermatoses immunes comme le lupus érythémateux, les maladies immunes bulleuses et les maladies du collagène, telle la sclérodermie ;
- Les stigmates de l'atrophie épidermique et/ou dermique induite par les corticostéroïdes locaux ou systémiques, ou toute autre forme d'atrophie cutanée,
- Les troubles de la cicatrisation, ou pour prévenir ou pour réparer les vergetures, ou encore pour favoriser la cicatrisation,
- Toute affection d'origine fongique au niveau cutané tel que le tinea pedis et le tinea versicolor,
- Les désordres de la pigmentation, tel l'hyperpigmentation, le mélasma, l'hypopigmentation ou le vitiligo ;
- Les états cancéreux ou précancéreux, cutanés ou muqueux comme les kératoses actiniques, la maladie de Bowen, les carcinomes in-situ, le kératoacanthome et les cancers cutanés comme le carcinome basocellulaire (BCC), le carcinome spinocellulaire (SCC) et les lymphomes cutanés tel que le lymphome T .

## Patentansprüche

1. Zusammensetzung vom Typ Emulsion Öl in Wasser, umfassend
- eine Fettphase, umfassend:
3"-tert-Butyl-4'-(2-hydroxy-ethoxy)-4"-pyrrolidin-1-yl-[1,1';3',1"]-terphenyl-4-carbonsäure; zumindest ein Hauptlösungsmittel von 3"-tert-Butyl-4'-(2-hydroxy-ethoxy)-4"-pyrrolidin-1-yl-[1,1';3',1"]-terphenyl-4-carbonsäure, ausgewählt aus Benzylalkohol, Laureth-4, Phenoxyethanol, Propylenglycolmonocaprylat, Pentylenglycol, Dimethylisosorbid und Mischungen davon, und zumindest ein Co-Lösungsmittelöl von 3"-tert-Butyl-4'-(2-hydroxy-ethoxy)-4-pyrrolidin-1-yl-[1,1';3',1"]-terphenyl-4-carbonsäure, ausgewählt aus Caprylic-/Capric-Triglyceriden, Süßmandelöl, Propylenglycolmonocaprylat, Propylenglycollaurat, Sorbitansesquioleat, Diisopropyladipat, PPG-15-Stearylether, PEG-6-Ester und Aprikosenkernöl und Mischungen davon, und
- eine wässrige Phase, umfassend zumindest ein Geliermittel, ausgewählt aus Polymeren pflanzlichen Ursprungs, Gummis, Pektinen, Zellulose und Derivaten davon, Polymeren mikrobiologischen Ursprungs wie Xanthangummi und gelbildenden Polymeren synthetischen Ursprungs.

2. Zusammensetzung nach Anspruch 1, **dadurch gekennzeichnet, dass** das Geliermittel ausgewählt ist aus:
- Acrylaten/C10-30-Alkylacrylat-Crosspolymer, vertrieben unter dem Namen Pemulen TR-1 oder Pemulen TR-2,
- Geliermitteln aus der Familie der Polyacrylamide wie die Mischung aus Natriumacrylamid/Acryloyldimethyltaurat-Copolymer/Isohexadecan/Polysorbat 80, vertrieben unter dem Namen Simulgel 600PHA,
- der Mischung Polyacrylamid/Isoparaffin C13-14/Laureth-7, vertrieben unter dem Namen Sepigel 305,
- Carbomeren, vertrieben unter den Namen Ultrez 20^{®}, Ultrez 10^{®}, Carbopol 1382^{®} oder Carbopol ETD2020NF^{®}, Carbopol 981 oder Carbopol 980,
- Polysacchariden mit, als nicht einschränkende Beispiele, Xanthangummi, wie Xantural180^{®}, Gellangummi, vertrieben unter dem Namen Kelcogel, Guargummi, Cellulose und Derivaten davon, wie mikrokristalline Cellulose und Natriumcarboxymethylcellulose, vertrieben unter dem Namen Avicel CL-611, Hydroxypropylmethylcellulose, insbesondere das Produkt, das unter dem Namen Methocel E4M Premium vertrieben wird, oder Hydroxyethylcellulose, insbesondere das Produkt, das unter dem Namen Natrosol HHX 250^{®} vertrieben wird, Natriumcarboxymethylcellulose, insbesondere Blanose Cellulosegummi 7F,
- der Familie der Aluminium-Magnesium-Silikate wie Veegum K,
- der Familie von Acrylpolymeren, die an hydrophobe Ketten gekoppelt sind, wie PEG-150/Decyl/SMDI-Copolymer, vertrieben unter dem Namen Aculyn 44,
- der Familie der modifizierten Stärken wie modifizierte Kartoffelstärke, vertrieben unter dem Namen Structure Solanace, oder Mischungen davon,
- der Familie der Carrageene, insbesondere unterteilt in vier Hauptfamilien: κ, λ, β, ω wie Viscarin^{®} und Gelcarin^{®}.

3. Zusammensetzung nach einem der vorhergehenden Ansprüche, **dadurch gekennzeichnet, dass** das Hauptlösungsmittel Phenoxyethanol ist.

4. Zusammensetzung nach einem der vorhergehenden Ansprüche, **dadurch gekennzeichnet, dass** sie ferner einen oder mehrere Zusatzstoffe enthält, ausgewählt aus:
- einem oder mehreren Konservierungsmitteln, ausgewählt aus Methylparaben, Propylparaben, Benzalkoniumchlorid, Phenoxyethanol, vertrieben unter dem Namen Phenoxetol, Benzylalkohol, vertrieben unter dem Namen Benzylalkohol, Kaliumsorbat, vertrieben unter dem Namen Kaliumsorbat, Benzoesäure, 2-Brom-2-Nitropropan-1,3-Diol, vertrieben unter dem Namen Bronopol, Chlorhexidin, Chlorhexidindigluconat, Chlorkresol und Derivaten davon, Ethylalkohol und Diazolidinylharnstoff,
- einem oder mehreren Chelatbildnern wie EDTA (Ethylendiamintetraessigsäure) und Derivaten oder Salzen davon, Dihydroglycerin, Zitronen- und Weinsäure, Gluconolacton, vertrieben unter dem Namen Glucono-delta-lactone SG, oder Mischungen davon,
- einem oder mehreren Antioxidantien wie Vitamin E und Derivaten davon, wie DL alpha Tocopherol oder Tocopherolacetat; Vitamin C und Derivaten davon, wie Ascorbylpalmitat, Butylhydroxytoluol, vertrieben unter dem Namen Nipanox BHT,
- einem oder mehreren beruhigenden und/oder reizhemmenden Mitteln wie PPG-12/SMDI-Copolymer unter dem Handelsnamen Polyolprepolymer-2, Glycyrrhetinsäure oder Derivaten davon, wie zum Beispiel Enoxolon, Hyaluronsäure als solche oder in ihrer Natriumhyaluronatform, vertrieben unter dem Handelsnamen HYAL. NA PWD PH 15-51-45, Allantoin, vertrieben unter dem Namen RONACARE ALLANTOINE.

5. Zusammensetzung nach einem der vorhergehenden Ansprüche, **dadurch gekennzeichnet, dass** sie ferner ein Mineralöl enthält.

6. Zusammensetzung nach einem der vorhergehenden Ansprüche, **dadurch gekennzeichnet, dass** sie ferner ein Feuchthaltemittel enthält.

7. Zusammensetzung nach einem der vorhergehenden Ansprüche, **dadurch gekennzeichnet, dass** sie ferner ein Silikonöl enthält.

8. Zusammensetzung nach einem der vorhergehenden Ansprüche, **dadurch gekennzeichnet, dass** sie ferner Ethanol enthält.

9. Zusammensetzung nach einem der Ansprüche 1 bis 8, **dadurch gekennzeichnet, dass** sie die folgenden Inhaltsstoffe enthält:
0,00001 bis 1 Gew.-% und bevorzugt 0,0001 bis 0,1 Gew.-% und bevorzugter 0,001 bis 0,1 Gew.-% der Verbindung von 3"-tert-Butyl-4'-(2-hydroxy-ethoxy)-4"-pyrrolidin-1-yl-[1,1';3',1"]-terphenyl-4-carbonsäure,
0,005 bis 10 Gew.-% und bevorzugt 1 bis 5 Gew.-% Geliermittel wie in Anspruch 1 definiert,
0,2 bis 5 Gew.-% und bevorzugt 0,5 bis 2 Gew.-% Hauptlösungsmittel wie in Anspruch 1 definiert,
0,5 bis 50 Gew.-% und bevorzugt 4 bis 30 Gew.-% Co-Lösungsmittelöl(e) wie in Anspruch 1 definiert,
und optional:
0 bis 20 Gew.-% und bevorzugt 0 bis 5 Gew.-% Mineralöl(e),
0 bis 50 Gew.-% und bevorzugt 5 bis 35 Gew.-% Polyol(e),
0 bis 10 Gew.-% und bevorzugt 0 bis 4 Gew.-% Silikonöl(e),
0 bis 5 Gew.-% und bevorzugt 0,01 bis 2 Gew.-% Konservierungsmittel,
0 bis 30 Gew.-% und bevorzugt 0 bis 10 Gew.-% Ethanol,
0 bis 15 Gew.-% und bevorzugt 0,1 bis 10 Gew.-% Additiv(e).

10. Zusammensetzung nach einem der vorhergehenden Ansprüche, **dadurch gekennzeichnet, dass** die maximale Menge an Wirkstoff , die 16 Stunden nach Anwendung in die Dermis und die Epidermis absorbiert wird, zwischen 6 ng/cm² und 19 ng/cm² liegt.

11. Zusammensetzung nach Anspruch 10, **dadurch gekennzeichnet, dass** die maximale Menge an Wirkstoff, die 16 Stunden nach Anwendung in die Dermis und die Epidermis absorbiert wird, zwischen 6,8 ng/cm² und 10,6 ng/cm² liegt.

12. Zusammensetzung nach einem der vorhergehenden Ansprüche, **dadurch gekennzeichnet, dass** die maximale Menge an Wirkstoff, die in die Epidermis absorbiert wird, zwischen 3 und 10 Stunden nach Anwendung erhalten wird.

13. Zusammensetzung nach einem der Ansprüche 1 bis 12 für ihre Verwendung als Arzneimittel.

14. Zusammensetzung nach einem der Ansprüche 1 bis 13 für ihre Verwendung bei der Behandlung von einer oder mehreren der folgenden Pathologien:
- dermatologischen Erkrankungen im Zusammenhang mit einer Keratinisierungsstörung bezüglich Zelldifferenzierung und - proliferation, insbesondere zur Behandlung von Akne vulgaris, komedonaler Akne, polymorpher Akne, Rosazea, nodulozystischer Akne, Conglobata, Altersakne, sekundärer Akne wie Sonnen-, Arzneimittel- oder Berufsakne;
- Verhornungsstörungen, insbesondere Ichthyose, ichthyosiformen Zuständen, lamellärer Ichthyose, Morbus Darrier, palmoplantarer Keratodermie, Leukoplakie, Pityriasis rubra pilaris und leukoplasiformen Zuständen, Haut- oder Schleimhautflechten (bukkal);
- dermatologischen Erkrankungen mit einer entzündlichen immunallergischen Komponente, mit oder ohne Zellproliferationsstörung, und insbesondere allen Formen der Psoriasis, ob kutan, mukosal oder ungual, und sogar psoriatischer Arthritis oder sogar atopischer Dermatitis und der verschiedenen Formen von Ekzemen;
- Hauterkrankungen aufgrund einer Einwirkung von UV-Strahlung sowie zur Reparatur oder Bekämpfung der Hautalterung, ob lichtbedingt oder chronologisch, oder zur Verringerung von Pigmentierungen und aktinischen Keratosen oder jeglichen Pathologien, die mit chronologischer oder aktinischer Alterung verbunden sind, wie Xerose, Pigmentflecken und Falten;
- jeglichem Zustand im Zusammenhang mit gutartigen dermalen oder epidermalen Proliferationen, unabhängig davon, ob sie viralen Ursprungs sind oder nicht, wie gewöhnliche Warzen, flache Warzen, Molluscum contagiosum und Epidermodysplasia verruciformis, orale oder floride Papillomatose;
- dermatologischen Erkrankungen wie Immundermatosen wie Lupus erythematodes, bullösen Immunerkrankungen und Kollagenerkrankungen wie Sklerodermie;
- Stigmata der epidermalen und/oder dermalen Atrophie, die durch lokale oder systemische Kortikosteroide oder jegliche andere Form der Hautatrophie induziert werden,
- Heilungsstörungen oder zur Vorbeugung oder Reparatur von Dehnungsstreifen oder zur Förderung der Heilung,
- Pilzerkrankungen der Haut wie Tinea pedis und Tinea versicolor,
- Pigmentstörungen wie Hyperpigmentierung, Melasma, Hypopigmentierung oder Vitiligo;
- krebsartigen oder präkanzerösen Haut- oder Schleimhauterkrankungen wie aktinischen Keratosen, Morbus Bowen, Karzinomen in situ, Keratoakanthom und Hautkrebs wie Basalzellkarzinom (BCC), Plattenepithelkarzinom (SCC) und kutanen Lymphomen wie T-Zell-Lymphom.

## Claims

1. An oil-in-water emulsion-type composition comprising
- a fatty phase comprising:
3"-tert-butyl-4'-(2- hydroxy-ethoxy)-4"-pyrrolidin-1-yl-[1,1';3',1"]-terphenyl-4-carboxylic acid;
at least one principal solvent of 3"-tert-butyl-4'-(2- hydroxy-ethoxy)-4"-pyrrolidin-1-yl-[1,1';3',1"]-terphenyl-4-carboxylic acid selected from among benzyl alcohol, laureth-4, phenoxyethanol, propylene glycol monocaprylate, pentylene glycol, dimethyl isosorbide and mixtures thereof, and at least one co-solvent oil of 3"-tert-butyl-4'-(2- hydroxy-ethoxy)-4"-pyrrolidin-1-yl-[1,1';3',1"]-terphenyl-4-carboxylic acid selected from among the caprylic/capric triglycerides, sweet almond oil, propylene glycol monocaprylate, propylene glycol laurate, sorbitan sesquioleate, diisopropyl adipate, PPG-15 stearyl ether, ester of PEG-6 and apricot kernel oil, and mixtures thereof, and
- an aqueous phase comprising at least one gelifying agent selected from polymers of plant origin, gums, pectins, cellulose and its derivatives, polymers of microbiological origin, such as xanthan gum, and gelifying polymers of synthetic origin.

2. The composition according to claim 1, **characterised in that** the gelifying agent is selected from:
- the Acrylates/C10-30 Alkyl Acrylate Crosspolymer sold under the name of Pemulen TR-1 or Pemulen TR-2,
- the gelifying agents in the family of polyacrylamides, such as the mixture Sodium acrylamide/acryloyldimethyltaurate copolymer / isohexadecane / polysorbate 80 sold under the name of Simulgel 600PHA
- the mixture polyacrylamide/isoparaffin C13-14/laureth-7 sold under the name of Sepigel 305,
- the carbomers sold under the name of Ultrez 20^{®}, Ultrez 10^{®}, Carbopol 1382^{®} or Carbopol ETD2020NF^{®}, Carbopol 981 or even Carbopol 980,
- the polysaccharides with, by way of non-exhaustive example, xanthan gum, such as Xantural180^{®}, gellan gum sold under the name of Kelcogel, guar gum, cellulose and its derivatives, such as microcrystalline cellulose and carboxymethyl cellulose of sodium sold under the name of Avicel CL 611 hydroxypropylmethylcellulose, in particular the product sold under the name of Methocel E4M premium or hydroxyethylcellulose , in particular the product sold under the name of Natrosol HHX 250^{®}, sodium carboxymethylcellulose, in particular Blanose cellulose gum 7F
- the family of aluminium magnesium silicates, such as Veegum K,
- the family of acrylic polymers linked to hydrophobic chains such as PEG-150/decyl/SMDI copolymer sold under the name of Aculyn 44,
- the family of modified starches such as modified potato starch, sold under the name of Structure Solanace or their mixtures,
- the family of carrageenans, in particular those distributed among four major families : κ, λ, β, ω such as the Viscarins^{®} and Gelcarins^{®}.

3. The composition according to any one of the preceding claims, **characterised in that** the principal solvent is phenoxyethanol.

4. The composition according to any one of the preceding claims, **characterised in that** it also contains one or more additives selected from:
- one or more preserving agents selected from among methyl parabene, propyl parabene, benzalconium chloride, phenoxyethanol sold under the name of phenoxetol, benzyl alcohol sold under the name of benzyl alcohol, potassium sorbate sold under the name of potassium sorbate, benzoic acid, 2-bromo-2-nitropropane-1,3-diol sold under the name of Bronopol, chlorohexidine, chlorohexidine digluconate, chlorocresole and its derivatives, ethyl alcohol and diazolidinyl urea,
- one or more chelating agents such as EDTA (ethylene diamine tetraacetic acid) and its derivatives or salts, dihydroglycerine, citric and tartaric acids, gluconolactone sold under the name of glucono-delta-lactone SG or mixtures thereof,
- one or more antioxidants such as vitamin E and its derivatives, such as DL alpha tocopherol or tocopherol acetate; vitamin C and its derivatives, such as Ascorbyl Palmitate, Butylhydroxy toluene sold under the name of Nipanox BHT,
- one or more palliatives and/or anti-irritants such as PPG-12/SMDI copolymer under the commercial name of Polyolprepolymer-2, glycyrrhetinic acid or its derivatives, for example Enoxolone, hyaluronic acid as such or in its form of sodium hyaluronate sold under the commercial name of HYAL. NA PWD PH 15-51-45, allantoin sold under the name of RONACARE ALLANTOINE.

5. The composition according to any one of the preceding claims, **characterised in that** it also contains a mineral oil.

6. The composition according to any one of the preceding claims, **characterised in that** it also contains a moistening agent.

7. The composition according to any one of the preceding claims, **characterised in that** it also contains a silicone oil.

8. The composition according to any one of the preceding claims, **characterised in that** it also contains ethanol.

9. The composition according to any one of Claims 1 to 8, **characterised in that** it comprises the following ingredients:
from 0.00001% to 1% by weight and preferably from 0.0001 to 0.1 % by weight and more preferably from 0.001 to 0.1% by weight of 3"-tert-butyl-4'-(2- hydroxy-ethoxy)-4"-pyrrolidin-1-yl-[1,1';3',1"]-terphenyl-4-carboxylic acid
from 0.005 to 10 % by weight and preferably from 1 to 5% by weight of gelifying agent as defined in claim 1,
from 0.2 to 5% by weight and preferably from 0.5 to 2% by weight of principal solvent, as defined in claim 1
from 0.5 to 50% by weight and preferably from 4 to 30% by weight of co-solvent oil(s), as defined in claim 1,
and optionally:
from 0 to 20% by weight and preferably from 0 to 5% by weight of mineral oil(s),
from 0 to 50% by weight and preferably from 5 to 35% by weight of polyol(s),
from 0 to 10% by weight and preferably 0 to 4% by weight of silicone oil(s)
from 0 to 5% by weight and preferably from 0.01 to 2% by weight of preservative agent(s),
from 0 to 30% by weight and preferably from 0 to 10% by weight of ethanol,
from 0 to 15% by weight and preferably from 0.1 to 10% by weight of additive(s).

10. The composition according to any one of the preceding claims, **characterised in that** the maximum quantity of active ingredient absorbed in the dermis and epidermis 16 hours after application is between 6 ng/cm² and 19 ng/cm².

11. The composition according to Claim 10, **characterised in that** the maximum quantity of active ingredient absorbed in the dermis and epidermis 16 hours after application is between 6.8 ng/cm² and 10.6 ng/cm².

12. The composition according to any one of the preceding claims, **characterised in that** the maximum quantity of active ingredient absorbed in the epidermis is obtained between 3 and 10 hours after application.

13. The composition according to any one of Claims 1 to 12 for use as a medicament.

14. The composition according to any one of Claims 1 to 13 for use in the treatment of one or more of the following pathologies:
- dermatological complaints associated with a keratinisation disorder relating to cellular differentiation and proliferation, particularly for treating common acne, comedonic acne, polymorphic acne, rosacea acne, nodulocystic acne, conglobata acne, senile acne and secondary acnes such as solar, medicamentous or professional acne;
- keratinisation disorders, in particular ichtyoses, ichtyosiform conditions, lamellar ichtyosis, Darrier's disease, palmoplantar keratodermias, leukoplasias, pityriasis rubra pilaris and leukoplasiform conditions, cutaneous or mucous (oral) lichen;
- dermatological disorders with an inflammatory immune-allergic component, with or without cellular proliferation disorder, and in particular all forms of psoriasis, whether cutaneous, mucous or ungueal, and even psoriasic rheumatism, or atopical dermatitis and the different forms of eczema;
- cutaneous disorders due to exposure to UV radiation, as well as to repair or control ageing of the skin, whether photo-induced or chronological, or to reduce pigmentations and actinic keratoses, or all pathologies associated with chronological or actinic ageing, such as xerosis, pigmentations and wrinkles;
- any condition associated with benign dermal or epidermal proliferations, whether or not of viral origin, such as common warts, flat warts, molluscum contagiosum and verruciform epidermodysplasia, oral or florid papillomatoses;
- dermatological complaints such as immune dermatoses such as erythematous lupus, bullous immune diseases and collagenic diseases such as sclerodermia;
- stigmata of epidermal and/or dermal atrophy induced by local or systemic corticosteroids, or any other form of cutaneous atrophy;
- healing complaints, or to prevent or repair stretch marks, or even to promote healing;
- any disorder of fungal origin in the cutaneous region, such as tinea pedis and tinea versicolor;
- pigmentation disorders such as hyperpigmentation, melasma, hypopigmentation or vitiligo;
- cancerous or pre-cancerous, cutaneous or mucous conditions such as actinic keratoses, Bowen's disease, carcinomas in-situ, keratoacanthoma and skin cancers such as basocellular carcinoma (BCC), spinocellular carcinoma (SCC) and cutaneous lymphomas such as T lymphoma.
